# EUROPEAN PATENT SPECIFICATION

(11) **EP 0 765 336 B1**
(45) Date of publication and mention of the grant of the patent: **02.05.2002**
(21) Application number: 95923887.4
(22) Date of filing: 14.06.1995
(51) Int. Cl.: C07H 3/06, C07C 35/14, C07C 41/03, C07D 317/46, C07D 203/26

(54) **SYNTHESIS OF CONDURITOL EPOXIDES AND AZIRIDINES AND METHODS OF USING SUCH TO SYNTHESIZE HIGHER DISACCHARIDES**
SYNTHESE VON CONDURITOLEPOXIDEN UND AZIRIDINEN UND VERFAHREN ZUR SYNTHESE HÖHERER DISACCHARIDE UND DEREN VERWENDUNG
SYNTHESE D'EPOXYDES DE CONDURITOL ET D'AZIRIDINES ET LEUR UTILISATION POUR LA SYNTHESE DE DISACCHARIDES SUPERIEURS

(30) Priority: 17.06.1994 US 261586
(43) Date of publication of application: 02.04.1997
(73) Proprietor: VIRGINIA TECH INTELLECTUAL PROPERTIES, INC., Blacksburg, VA 24060 (US)
(72) Inventor: HUDLICKY, Tomas, Blacksburg, VA 24060 (US)
(74) Representative: Kiddle, Simon John
(86) International application number: US9507601
(87) International publication number: WO9535303

(56) References cited:
- J. AM. CHEM. SOC., vol. 117, 1995 pages 3643-4, T. HUDLICKY ET AL. 'First enantioselective total synthesis of (+)- pancratistatin: an unusual set of problems'
- J. ORG. CHEM., vol. 59, 1994 pages 4037-9, T. HUDLICKY ET AL. 'Oxa- and azabicyclo(4.1.0)heptenes as new synthons for C-disaccharide and alkaloid synthesis. Reactivity trends with carbon nucleophiles'
- SYNLETT, 1992 pages 291-2, S.V. LEY ET AL. 'Microbial oxidation in synthesis: preparation of pseudo-alpha-D-glucopyranose from benzene' cited in the application
- TETRAHEDR. LETT., vol. 29, 1988 pages 5305-8, S.V. LEY ET AL. 'Microbial oxidation in synthesis: preparation from benzene of the cellular secondary messenger myo-inositol-1,4,5-triphosphate (IP3) and related derivatives'
- J. ORG. CHEM., vol. 58, 1993 pages 985-7, T. HUDLICKY ET AL. 'Rational design of aza sugars via biocatalysis: mannojirimycin and other glycosidase inhibitors'
- J. ORG. CHEM., vol. 57, 1992 pages 5861-8, K. RAMESH ET AL. 'Synthesis of hydroxylated cyclohexenyl- and cyclohexanyladenines as potential inhibitors of S-adenosylhomocysteine hydrolase'
- CHEMICAL ABSTRACTS, vol. 121, no. 15, 1994 Columbus, Ohio, US; abstract no. 180056w, H. SECEN ET AL. 'Stereospecific synthesis of conduramine-F4 and conduritol-F' page 1127; & SYNTH. COMMUN., vol. 24, 1994 pages 2103-8,
- J. CHEM. SOC. PERKIN TRANS. 1, 1991 pages 2907-17, T. HUDLICKY ET AL. 'Biocatalysis as the strategy of choice in the exhaustive enantiomerically controlled synthesis of conduritols' cited in the application
- ANGEW. CHEM. INT. ED., vol. 33, 1994 pages 652-4, T. IBUKA ET AL. 'A novel route to diastereomerically pure (E)-alkene dipeptide isosters from beta-aziridinyl-alpha,beta-enoates by treatment with organocopper reagents'
- CHEM. BER., vol. 121, 1988 pages 757-80, H. PRINZBACH ET AL. 'cis/trans-(1.1.1)-O,N,C-Tris-sigma-homobe nzole'

## Description

### FIELD OF THE INVENTION

This invention relates to novel processes for the synthesis of certain conduritol epoxides and aziridines and the use of these epoxides and aziridines to synthesize various or higher disaccharides such as conjugates of cyclitols and/or carbohydrates and their carbocyclic analogs (C-analogs), as well as various heteroatom-linked conjugates such as N-, S- or O-linked conjugates. This invention also relates to certain novel compounds useful as synthons and/or as therapeutic agents useful in treating various disease in a mammal.

### BACKGROUND OF THE INVENTION

The expression of arene cis-diols was originally discovered and described by Gibson 24 years ago (Gibson, D. T.; Hensley, M.; Yoshioka, H.; Mabry, J. J. *Biochemistry*, 1970, 9, 1626). Since that time, use of such arene *cis*-diols in enantiocontrolled synthesis of oxygenated compounds has gained increasing acceptance by those skilled in the art. Many examples of their applications to the total synthesis of carbohydrates, cyclitols, and oxygenated alkaloids can be found in the literature. For example, T. Hudlicky *et al.* (*J. Org. Chem.* **58**(5), 985-7, 1993) describe a synthesis of mannojirimycin which employs, as an intermediate step, stereoselective ring-opening of an optically active cyclohexadiene epoxide by azide ion (N₃⁻). The epoxide was prepared by enzymatic hydrolysis of chlorobenzene to the arene cis-diol, followed by protection of the diol and epoxidation. However, much of the work done within this area has been with the more traditional approach of attaining optically pure compounds from the carbohydrate chiral pool. (Hanessian, S. in *Total Synthesis of Natural Products: The Chiron Approach*; Pergamon; Oxford, 1983).

The synthesis of glycoconjugates has attracted considerable attention recently [(a) Borman, S., *C&E News* 1994, 72, (9), 37; (b) Glycotechnology conference, San Francisco, 1993). Higher saccharides such as the gangliosides GM3 and sialyl Lewis x both of which are proposed to be involved in malignancy and intercellular adhesion, trans-cell membrane signal transduction and regulation of cell growth have been the subject of intense focus [(a) Danishefsky, S. J.; McClure, K. F.; Randolph. J. T.; Ruggeri. R. B. *Science* 1993. 260, 1307; (b) Liu, K. K.-C.; Danishefsky, S. J. *J. Am. Chem. Soc.* 1993, 115, 4933] by chemical as well as enzymatic means [Ichikawa, Y.; Lin, Y.-C.; Dumas, D. P.; Shen, G.-J.; Garcia-Junceda, E.; Williams, M. A.; Bayer, R.; Ketcham, C.; Walker, L. E.; Paulson, J. C.; Wong, C.-H. *Am. Chem. Soc.* 1992, 114, 9283]. The current chemical and/or enzymatic synthesis of these compounds is arduous at best giving rise to a growing body of high yielding, selective, chemical and enzymatic glycosidation methodologies [(a) Raghavan, S.; Kahne, D. A one-step synthesis of the ciclamycin trisaccharide. *J. Am. Chem. Soc.,* **1993**, 115, 1580 and the references therein.; (b) Frazer-Reid, B.; Zugan, W.; Andrews, W.; Skowronski, E. Torsional effects in glycoside reactivity: saccharide couplings mediated by acetate protecting groups. *J. Am. Chem. Soc.,* **1991**, 513, 1434.; (c) Frazer-Reid, B. *n*-Pentenyl glycosides in organic chemistry: a contemporary example of serendipity. *Synlett,* **1992**, 927.; (d) Veeneman, G.H.; van Leeuwen, S.H.; van Boom, J.H. An efficient thioglycoside-mediated formation of α-glycodidic linkages promoted by iodonium dicollidine perchlorate. *Tetrahedron Lett.,* **1990,** 31, 274.; (e) Kondo, H.; Achi, S.; Ichikawa, Y.; Halcomb, R.C.; Ritzen, H.; Wong, C.-H. Glycosyl phosphites as glycosidation reagents: scope and mechanism. *J. Org. Chem*., **1994,** 59, 864.; (f) Toshima, K.; Nozaki, Y.; Inokuchi, H.; Nakata, M.; Tatsuta, K.; Kinoshita, M. A new entry for the controlled synthesis of 2,6-dideoxy oligosaccharides. *Tetrahedron Lett.,* **1993,** 34, 1611]. However, these glycosidation methods are incompatible if the glycosidic donor is to be a carba-sugar and, therefore, would not be useful if a fully carbocyclic oligosaccharide analog were required. Thus, the carbocyclic analogs of these types of compounds are generally unattainable by currently available methods, unless long and arduous routes from carbohydrates are employed [see generally: Hanessian supra].

Whereas the carbocyclic analogs of simple sugars are known (Suami, T.; Ogawa, S. In *Advances in Carbohydrate Chemistry and Biochemistry;* Tipson, R. S.; Horton, D., Eds.; Academic: New York, 1990; Vol. 48, p 21; Ley, S. V.; Yeung, L. L. *Synlett* 1992, 291) an attempt at rational and exhaustive design of higher members is absent in the literature.

The present invention alleviates the problems associated with prior chemical or enzymatic synthesis for making cyclitol and carbohydrate conjugates and/or C-analogs thereof by providing methods and useful synthons such as cyclitol conjugates and aziridines which are specifically useful in pseudo-sugar couplings so that semi- and/or fully carba-analogs of carbohydrates can be prepared.

### SUMMARY OF THE INVENTION

Therefore, one aspect of this invention relates to biocatalytic methods of synthesis for various conjugates of cyclitols and carbohydrates and their C-, N-, S- or O-linked conjugates. Specifically the conjugates made by the present invention have the formula (1) 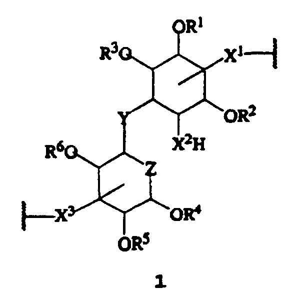 wherein:
X¹-X³ independently are CH₂, O, NH, or S;
Y is CH₂, O, NH, or S;
Z is CH₂, O, NH, or S; and
R¹-R⁶ independently are any alcohol protecting group.

Another aspect of the present invention relates to the synthesis and use of cyclitol epoxide **(2)** and cyclitol aziridine **(3)** as *electrophilic recipients* of other cyclitol or C-sugar units in the coupling reactions described herein.

Cyclitol epoxide **(2)** has the formula: 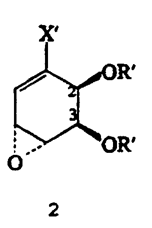 wherein:
X' is H, halogen, CN, alkyl (branched or unbranched C1-C5), aryl (substituted or unsubstituted aromatic) or a heteroatom (wherein the heteroatom may be alone or in a straight chain or ring structure); and
each R' is independently any alcohol protecting group; provided that the alcohols at C2 and C3 need not be protected with the same protecting group.

Aziridine **(3)** has the formula: 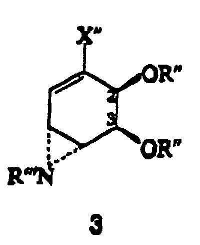 wherein:
X" is H, halogen, CN, alkyl (branched or unbranched C1-C5), aryl (substituted or unsubstituted aromatic) or a heteroatom (wherein the heteroatom may be alone or in a straight chain or ring structure);
each R" is independently any alcohol protecting group; provided that the alcohols at C2 and C3 need not be protected with the same protecting group; and
R''' is H, CBZ, tosyl or any substituted or unsubstituted arylsulfonic acid amide; benzyl or CO₂Me.

Compound **2**, its synthesis, and use as a synthon are described in commonly owned U.S. Patent Nos. 5,563,281, 5,578,738, 5,600,014, 5,602,263, and 5,602,287. Compound **(3)** is a novel aziridine and, therefore, another aspect of this invention relates to novel compounds of the formula **(3)**.

### DETAILED DESCRIPTION OF THE INVENTION

The present invention relies on understanding and controlling the sites of potential coupling of electrophiles **2** and **3** with nucleophiles. In the general structure **4** (encompassing such electrophiles), there are three possible pathways to 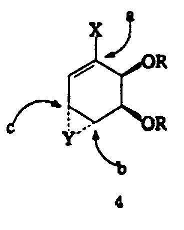 wherein:
X=H, Cl, Br, I, F
Y=O, NTs, NCBZ, NH
R=C(CH₃)₂, C=O, alkyl, acyl
open such electrophiles: **a**, **b**, and **c**. Path b is precluded by stereoelectronic effects, whereas path **a** and **c** are subject to normal S_{N} and S_{N'} arguments as described in the literature on the chemistry of vinyloxiranes. To date, the opening at sites **a** or **c** with carbon nucleophiles has been attained only with acetylide anion [Ley, S. V.; Yeung, L. L. *Synlett* 1992, 291-292]. The chemistry of vinylaziridines in general has been limited to their rearrangements to pyrrolines, which involves S_{N'} opening and reclosure with iodide [Hudlicky, T.; Reed, J. W. In *Comprehensive Organic Synthesis;* Paquette, L. A., Ed.; Pergamon: Oxford; Vol. 5, Chapter 8.1]. One example of a cuprate addition to vinyl aziridines has been recently reported. [Ibuka, T., et al. *Angew. Chem. Int. Ed. Engl*., 1994, 33, 652]. The preparation of **3** and its nucleophilic opening tendencies thus remained unknown until the present disclosure.

For the synthesis of glycoconjugates and especially their C-analogs that may offer complementary biological activities, it is desirable that the sites **a** and **c** be controlled equally well for heteroatom as well as carbon nucleophiles. For the synthesis of higher saccharides it is also desirable that such opening generate only one nucleophilic group at any given time so that it is available for the next reaction.

Thus the present invention describes the initial model studies necessary to establish the regio- and stereocontrol of nucleophilic opening for **2** and **3.** From these initial models, cyclitol and conduramine conjugates such as **5, 6,** and **7** as well as their further functionalization to compounds such as **8, 9,** and **10** have been made demonstrating that further attachment of protected cyclitol and sugar units is possible in a controlled manner.

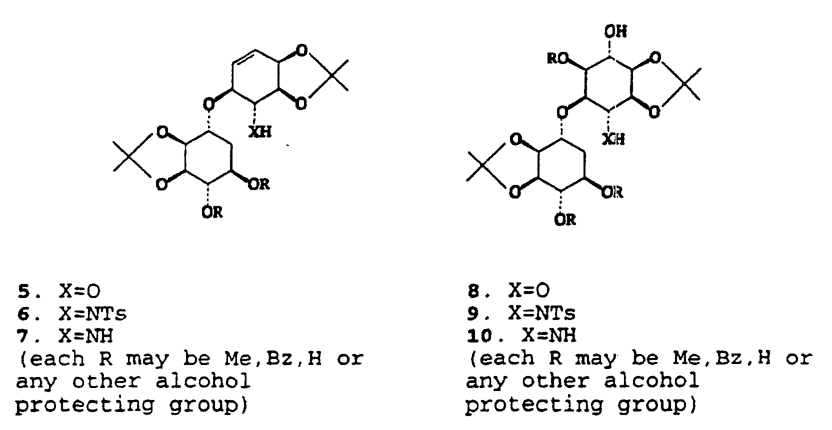

The demonstration of the present invention with regard to the synthesis of compounds **5-10** is merely illustrative of the present invention. Those skilled in the art will readily understand the implications of this methodology as it relates to preparation of any cyclitol or carbohydrate conjugate.

In addition, the preparation of fully carbocyclic analogs of the aforementioned compounds (**5-10**) are described herein, and their synthesis is envisioned by the present invention. Synthesis of these fully carbocyclic analogs rely on the coupling of an organometallic moiety such as **11,** which can be derived from dehydroshikimate **12** (Scheme 1).

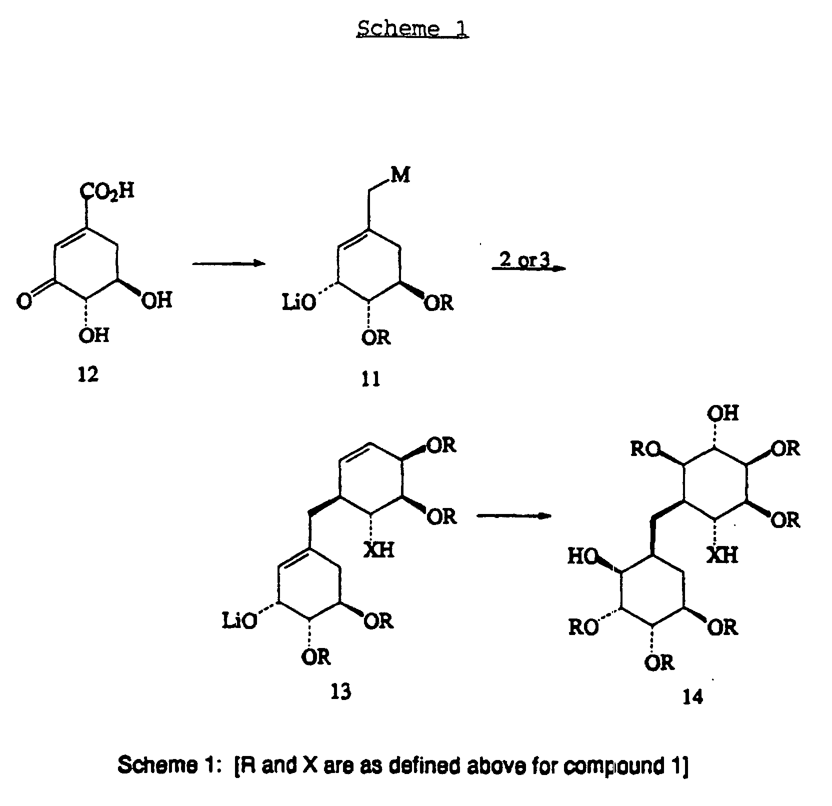

Although the present disclosure outlines model studies and reactivity trends of synthons **2** and **3** toward carbon nucleophiles, it also serves as a stereoelectronic model for the union of heteroatom nucleophiles with compounds **2** or **3** to produce higher saccharides and their C-analogs and various heteroatom-linked conjugates. Compounds such as **13** or its hydroxylated analog **14** [derived by methods disclosed in U.S. Patent No. 5,306,846 and in several publications: (a) hudlicky, T.; Reed, J. W. In *Advances in Asymmetric Synthesis:* A. Hassner, Ed.; JAI Press: Greenwich, CT, 1994; in press; (b) Hudlicky, T.; Rulin, F.; Tsunoda, T.; Luna, H.; Andersen, C.; Price, J. D. *Isr. J. Chem.* 1991, 31, 229; (c) Hudlicky, T.; Luna, H.; Olivo, H. F.; Andersen, C.; Nugent, T.; Price, J. D. *J. Chem Soc. Perkin Trans. 1* 1991, 2907] are inaccessible by methods currently employed for the synthesis of higher saccharides. Recent disclosures [(a) Ley, S. V.; Yeung, L. L. *Synlett* 1992, 997; (b) Reddy, K. K.; Falck, J. R.; Capdevila, J. *Tetrahedron Lett.* 1993, 34, 7869] indicate that such compounds may be useful as antidiabetic agents and in general cell-signalling mechanisms.

The currently disclosed invention capitalizes on some of the previously disclosed techniques of operationally simple provision of sugar or cyclitol units from arene *cis*-diols as is outlined in Scheme 2 [see also: (a) Hudlicky, T.; Mandel, M.; Rouden, J.; Lee, R. S.; Bachmann, B.; Dudding, T.; Yost, K. J.; Merola, J. S. *J. Chem. Soc., Perkin Trans. 1* 1994, 1553; (b) Hudlicky, T.; Rouden, J.; Luna, H.; Allen, S. *J. Am. Chem. Soc.* 1994, 116, 5099; (c) Hudlicky, T.; Olivo, H. F.; McKibben, B. *J. Am. Chem. Soc.* 1994, 116, 5108] and provides conceptual guide as to the oligomerization of these units as outlined in Scheme 3.

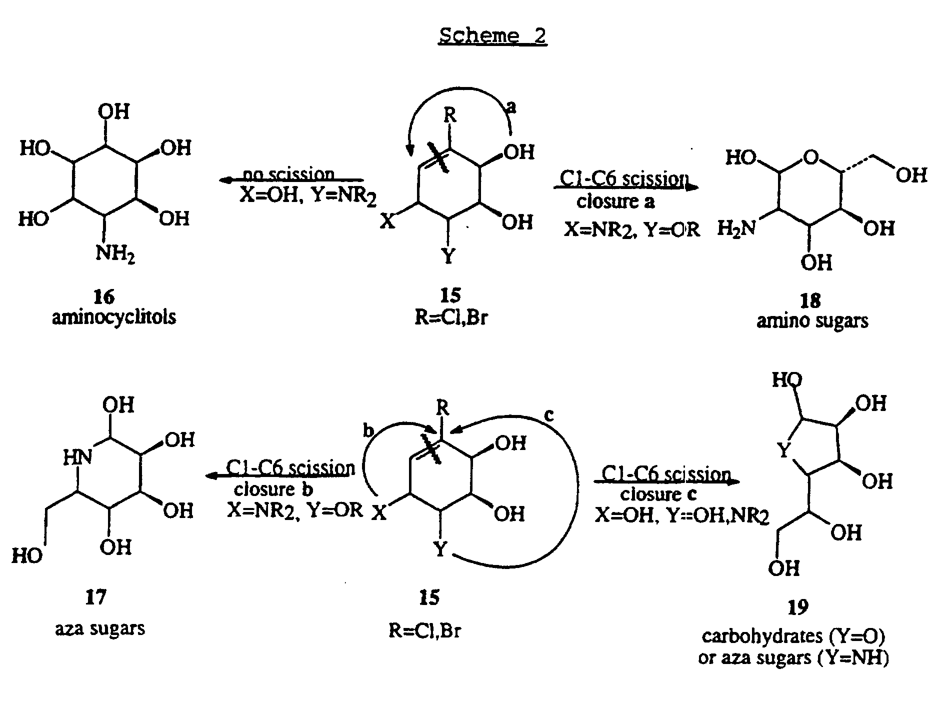

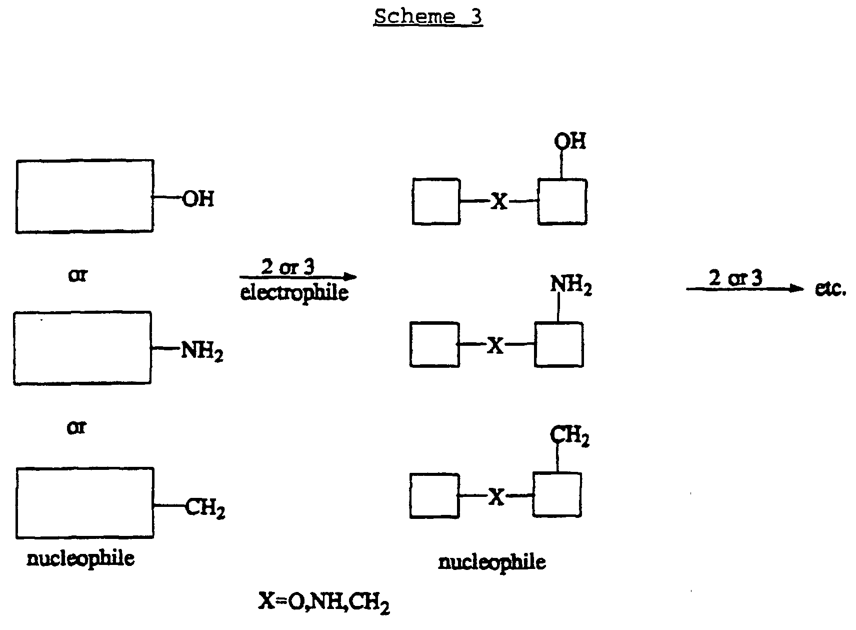

Scheme 3 illustrates in a very simplistic manner the overall impact of the present invention, whereby electrophiles such as **2** and **3** are opened at controlled sites, by heteroatom or carbon nucleophiles (under appropriate conditions) resulting in the coupling of the electrophile (conduritol epoxide or aziridine **2** or **3**) with the heteroatom or carbon atom which newly coupled nucleophile can repeatedly be coupled with similar electrophiles resulting ultimately in any number of carbohydrates or glycoconjugates.

Of additional significance in the current invention is the stepwise control of introduction of substituents made possible by differential protection as well as stereoelectronic differences between olefins in the cyclitol units. In other words, when protecting the electrophile with different groups and protecting the nucleophile with different groups, only one nucleophilic and electrophilic site will be available at a time. For example, as shown in Scheme 4, opening epoxide **20** with benzyl alcohol affords cyclitol **21** which is now disposed to act as a nucleophile when exposed to epoxide **22**. After coupling **21** and **22** there is only one hydroxyl in **23** and two olefins, which can be successfully functionalized to either *cis* or *trans* protected diols by methods disclosed in U.S. Patent No. 5,306,846, Hudlicky, T.; Reed, J. W. In *Advances in Asymmetric Synthesis*; A. Hassner, Ed.; JAI Press: Greenwich, CT, 1994; in press. For the synchesis of further conjugates this process may be repeated provided only one nucleophilic entity is present.

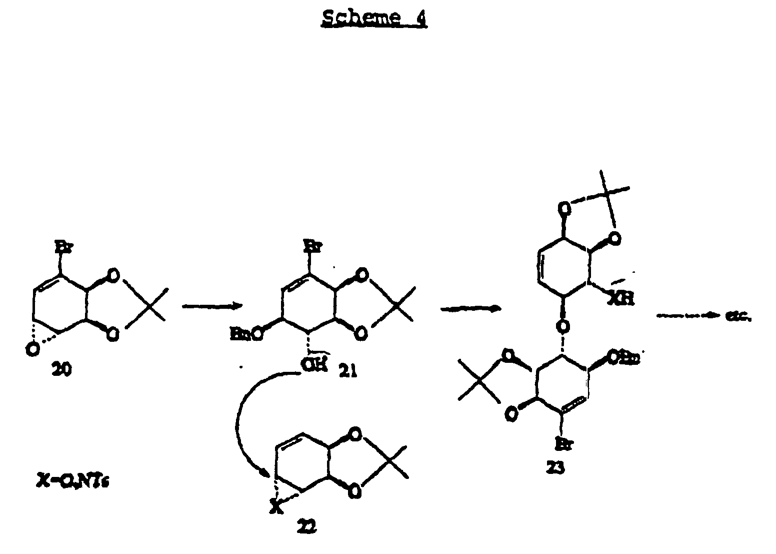

Further studies indicate that a methylcyclohexyl moiety can be effectively added to, with both epoxides and aziridines, to provide models for C-saccharide conjugates of the type shown below. This concept has been demonstrated by the synthesis of *gala*-quercitol-L-*chiro*-inositol conjugate starting from aromatic precursors, such as by Scheme 5 shown below.

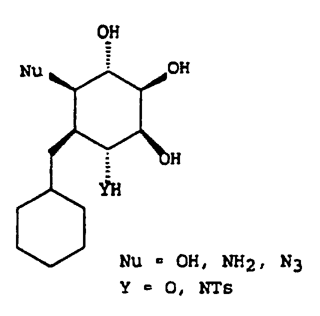

Reaction between the epoxide (Carless, H.A.J., *Tetrahedon Lett.,* 1993, 33, 6379) and the secondary alcohol (Hudlicky, T.; Luna, H.; Olivo, H.H.; Anderson, C.; Nugent, T.; Price, J.D.; *J. Chem. Soc. Perkin Trans*. 1, 1991, 2907; Hudlicky, T.; Mandel, M.; Rouden, J.; Lee, R.S.; Bachmann, B.; Dudding, T.; Yost, K.J.; Merola, J.S.; *J. Chem. Soc. Perkin Trans.* 1, 1994, 1553) both readily accessible from halobenzenes via microbial oxidation ((a) Gibson, D.T.; Koch, G.R.; Kallio, R.E.; *Biochemistry*; 1968, 7, 2653 (b) Gibson, D.T.; Hensley, M.; Yoshioka, H.; Mabry, J.J.; *Biochemistry,* 1970, 9, 1626)gave in the presence of boron trifluoride, the coupled adduct in 75% yield (Scheme 5). Predominant reaction of the vinyl epoxide at the allylic position follows observations in our laboratories (Hudlicky, T.; Fan, R.; unpublished results; (a) Hudlicky, T.; Konigsberger, K.; Xinrong, T.; *J. Org. Chem*. 1994, 59, 4037 (b) Hudlicky, T.; Rouden, J.; Luna, H.; Allen, S.; *J. Am. Chem. Soc*., 1994, 116, 5099) which indicate the general preference for nucleophiles to attack the allylic position of substrates such as the vinyl epoxide *syn* to the isopropylidene group. Treatment of the di-alkene (of Scheme 5) with osmium tetroxide, continuously recycled by 4-methyl morpholine *N*-oxide, gave the *bis*-hydroxylated species in good yield, which was subsequently converted to the polyoxygenated conjugate (*gala*-quercitol-L*-chiro*-inositol) under acidic catalysis.

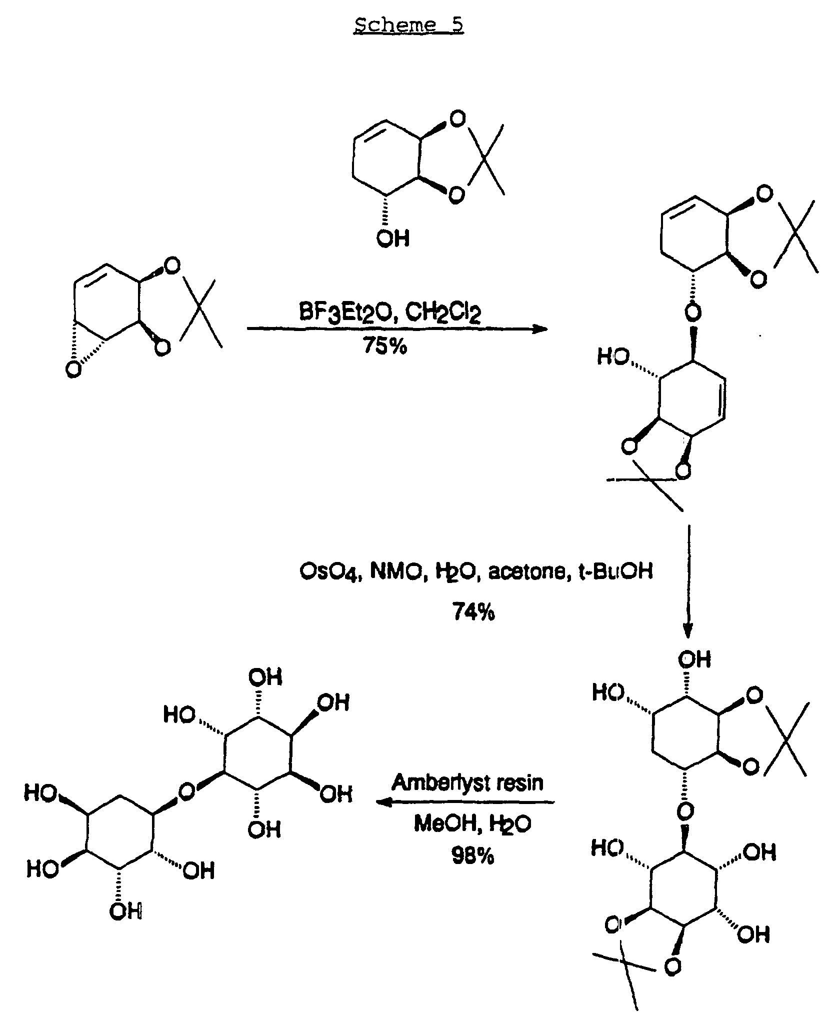

The current invention facilitates the design and synthesis of many higher cyclitol conjugates and their C-analogs unavailable by traditional methods. The combinational possibilities regarding substitution patterns, identity of protecting groups, stereochemistry and enantiomeric constitution are well understood to those skilled in the art. These parameters can be controlled at the stage of each monomeric unit.

As used in the present invention "suitable or appropriate solvents" include, but are not limited to, water, water miscible solvents such as dialkylketones with 2-4 carbon atoms, lower alcohols having 1-3 carbon atoms, cyclic ethers, and ethers with 2-6 carbon atoms, or mixtures thereof.

As used herein "reducing agent" includes, but is not limited to, a transition metal reagent, a hydride reagent or trialkylsilane such as tributyltinhydride or tris(trimethylsilyl) silane, sodium naphthalide, or sodium amalgam. These reducing agents may be used in combination with radical initiation agents such as UV light and/or AIBN or dibenzoylperoxide or a similar initiator.

As used herein "acid catalyst" includes, but is not limited to, mineral acids such as HCl; Lewis acids; organic acids such as *p*-toluenesulfonic acid; acid ion exchange resins such as Amberlyst 15, Amberlyst IR 118, Amberlite CG-50, Dowex 50 X 8-100 (all are commercially available from Aldrich), or similar acidic ion exchange resins.

As used herein "alkaline catalyst" includes, but is not limited to, alkaline metal hydroxide or alkaline earth metal hydroxides such as LiOH, NaOH, KOH, or Ba(OH)₂; carbonate or bicarbonate of alkaline metal such as Na₂CO₃ or K₂CO₃; Al₂O₃ or basic ion exchange resin such as Amberlite IRA-400, Amberlyst A26, Amberlyst A21, Dowex 1X2-200 or other ion exchange resins.

As used herein "an alcohol protecting group" includes, but is not limited to, H, acetate, acetonide, alkyl (C1-C5), aryl (any aromatic group substituted or unsubstituted), esters, ethers, silylethers, arylsulfonylamides, t-butyldimethylsilyl, benzyl, benzoates or any other alcohol protecting group known to those skilled in the art.

As used herein "an appropriate organometallic reagent" includes, but is not limited to, those described in Tables 1 and 2. Such reagents are commonly known to those skilled in the art. These are shown in Table 1 by the general formula RM where R is methyl, methylcyclohexyl, or phenyl, and M is Mg, Cu, Sn, Pd.

As used herein "carbon or heteroatom conjugate" means a C-, N-, O- or S-linked conjugate or analog of a given compound, such as a C-, N-, O- or S-linked analog of cyclitols and/or carbohydrates.

Suitable conditions, including solvents and temperatures are listed . in Tables 1 and 2 and include, but are not limited to, the types of solvents and temperature ranges respectively. Temperature ranges implies -100°C to +160°C.

### General Synthesis of Electrophiles 2 and 3

Epoxide **2** and aziridine **3** can be generated following the biocatalytic production of cis-diols derived from substituted benzenes. However, the coupling methods of the present invention will work regardless of the method used to make the electrophiles **2** and **3**. The synthesis of epoxide **2** is described in U.S. Patent 5,306,846 and U.S. Patent Nos. 5,563,281, 5,578,738, 5,600,014, 5,602,263, and 5,602,287 and Hudlicky, T., Rulin, F., Tsunoda, T., Luna, H., Andersen, C., Price, J. D. *Isr. J. Chem.* 1991. 31, 229. The aziridine is a novel compound and its synthesis is described both generally and in detail in the Experimental section herein. General methods for aziridine formation are found in Yamada, Y., Yamamoto. T., Okawara, M. *Chem. Lett.* 1975, 361; Evans, D. A., Faul, M. M., Bilodeau, M. T. *J. Org. Chem.* 1991, 56, 6744; and Evans, D. A., Faul, M. M., Bilodeau, M. T., Anderson. B. A., Barnes, D. M. *J. Am. Chem. Soc.* 1993, 115, 5326.

### Use of Synthons 2 and 3

Compounds such as **14**, **23** and **24** are generated by exposing either epoxide **2** or aziridine **3** to nucleophilic monomers such as **11** derived from dehydroshikimate. These initial targets are identified and fully deprotected to obtain corresponding compounds containing all free hydroxyl or amino groups. These compounds may be used in the treatment of diabetes or other cell-signalling mediated diseases.

Partially protected dimers such as **14**, **23** and **24** may then be used in further coupling to generate trimers which can subsequently be coupled to form tetramers. etc. The details of methods available for the coupling of sugars or cyclitol monomers are provided below.

Table 1 lists the major products obtained thus far by opening of epoxides **2a** and **2b** with organometallic reagents of the general formula RM where R is methyl, methylcyclohexyl, or phenyl, and M is Mg, Cu, Sn, Pd. The Table is not intended to limit the present invention, particularly since these reactions are disclosed to serve as *model systems* for C-disaccharide synthesis, as, for example, opening of an epoxide **(2a)** with methyl organometallics to yield **25.** Compound **25** can be used for the synthesis of methylquercitol (deoxy-C-mannose) derivative **45** as shown below.

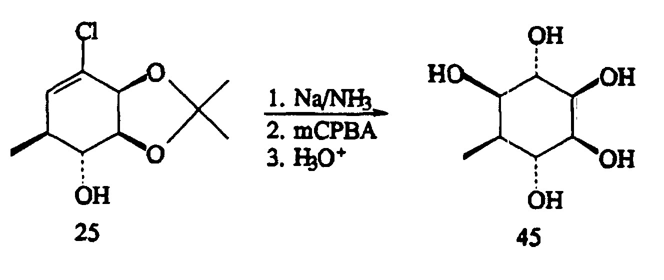

By the same token, the use of methylcyclohexyl residues provides C-disaccharide model Compound **46** by the reaction shown below:

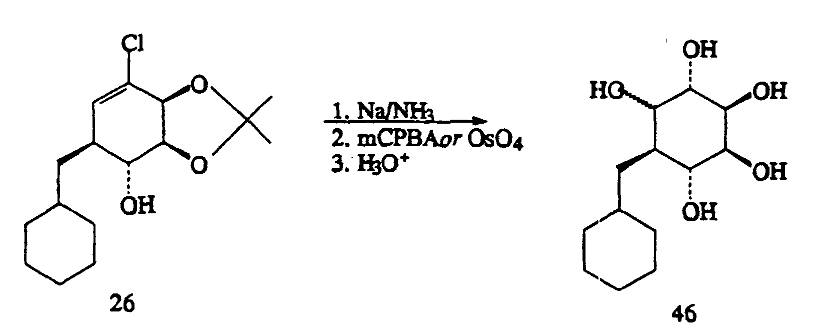

Further application of the coupling methods of this invention is illustrated by the reaction below showing an azido or amino derivative of **46**, namely **47** and **48** respectively, available from **26** by the sequences shown:

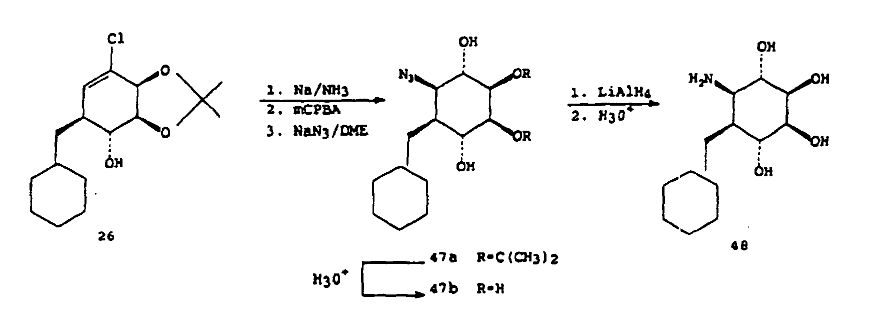

A trisaccharide can be made by the coupling methods described herein, for example, **49** is available from **27** by full deprotection. It should be noted that isomers **32** and **33** (shown in Table 1) are ideally suited as disaccharide models also, albeit in different configurations than the C-mannose **44**.

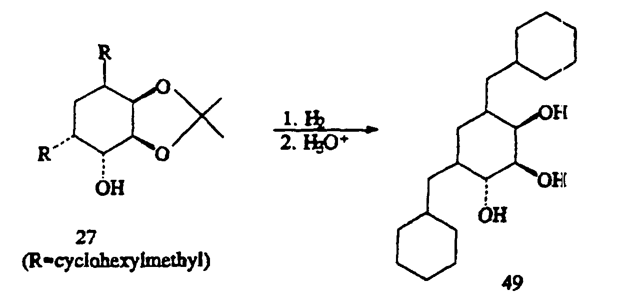

Table 2 lists the major products obtained thus far by opening aziridines **3a** and **3b.** Table 2 is not intended to limit the present invention, particularly since these reactions are provided as a model system for coupling reactions. One skilled in the art will recognize various embodiments of the present invention such as those described herein.

Aminoinositol compounds of the type **50** are accessible as shown in the examples below:

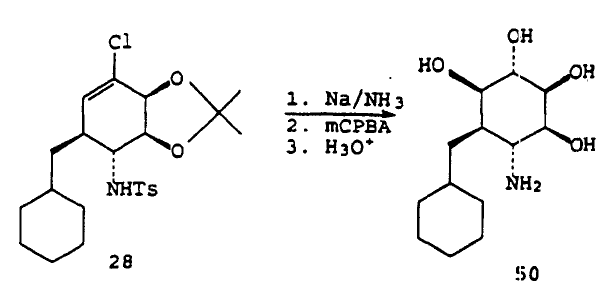

Phenyl (CuCN) cuprate was used to form product **43** in an improved yield of 49%. It is contemplated that using the methods of the present invention the compound pancratistatin **53**, a known anticancer agent, can be made from the known compound **51** (C.H. Heathcock, et al. *Tetrahedron Lett.,* 1992, 6775) based on the conversion of compound **51** to **52** as shown.

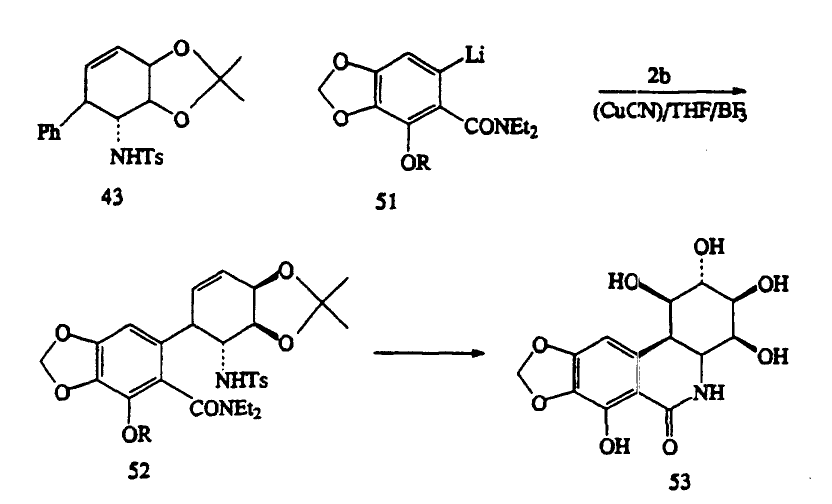

It is further contemplated that the coupling methods described herein can be employed in pseudo-sugar coupling such that semi- or fully carba-analogs of the general type **55** or **57** or other heteroatom conjugates of **54** and **56** could be made.

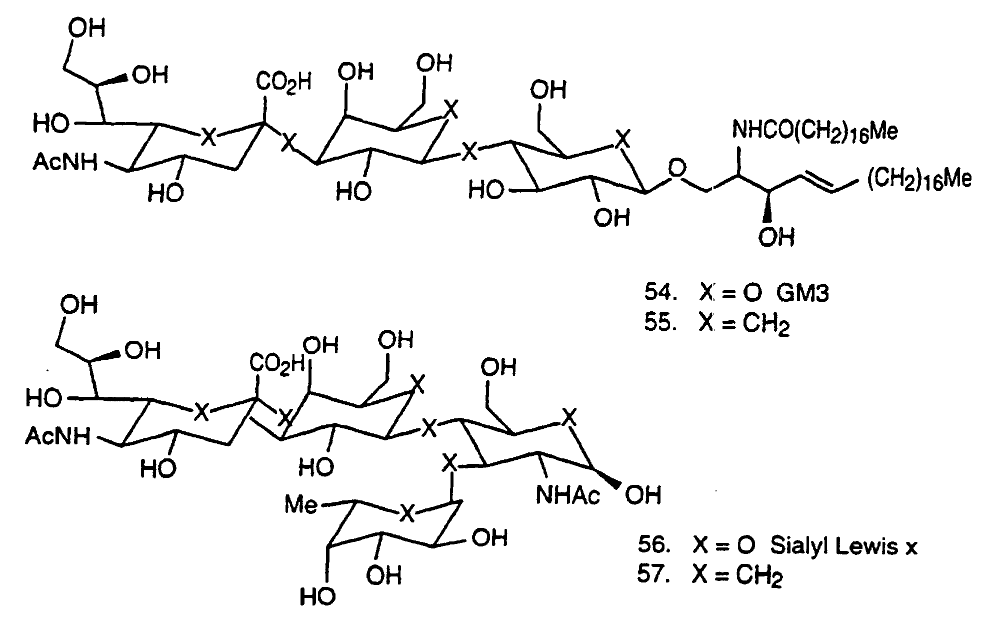

The ganglioside GM3 **54** and its metabolic precursors are important starting points for the biosynthesis of other gangliosides. Gangliosides in general have been proposed as being involved in malignancy and intercellular adhesion, trans-cell membrane signal transduction and regulation of cell growth. GM₃ itself is known to be active in governing epidermal growth factor and platelet-derived growth factor receptors, and importantly is found in much higher concentrations in tumor cells. Sialyl Lewis x **56** is an important cellular surface oligosaccharide involved in cellular adhesion processes and also found in increased amounts in patients suffering from breast, pancreatic and gastrointestinal cancer. The interest in these two structurally similar oligosaccharides has stimulated several chemical and enzymatic total syntheses.

For these reasons there should be great interest in the synthesis of carba-oligosaccharide analogs of GM₃ or sialyl Lewis x. We propose that access to these types of semi- or fully-carbocyclic mimics should be attainable by coupling nucleophilic carba-sugars with standard glycosidic donors or electrophilic carba-sugar epoxides and aziridines.

The precise choice of O-atom replacement within GM3 (see **55** above) will await the synthesis of the first such derivative containing O-linked carba-sugars and its biological evaluation. Following this result, rational thought and design will be applied to the definition of the next specific target. We believe that the demonstration of the capability to synthesize such carba-analogs in a controlled manner will open the way for the preparation of any isomer or isostere of these and other saccharides.

Exemplary reactions for vinyloxiranines and vinylaziridines are shown in Tables 1 and 2 respectfully.

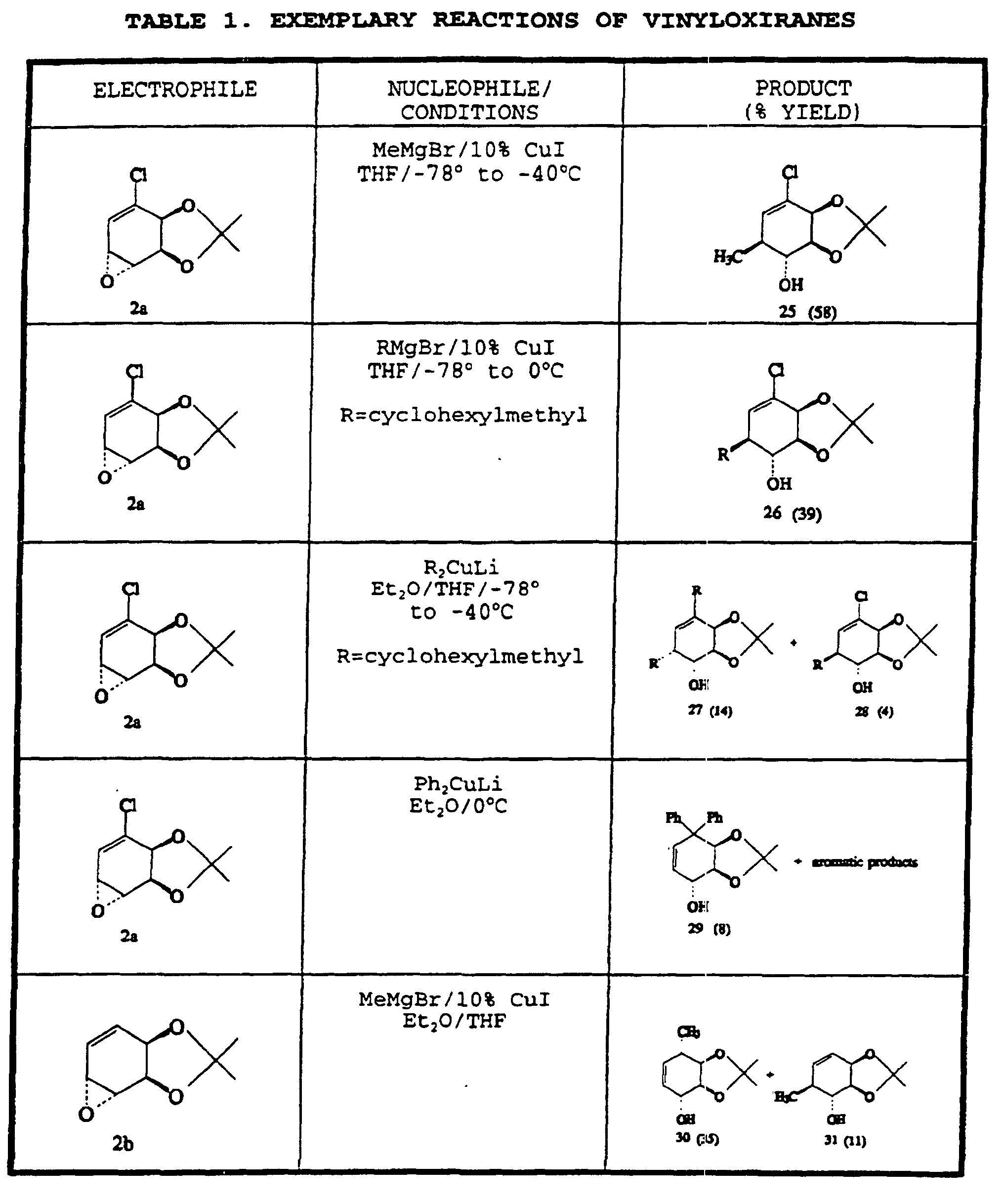

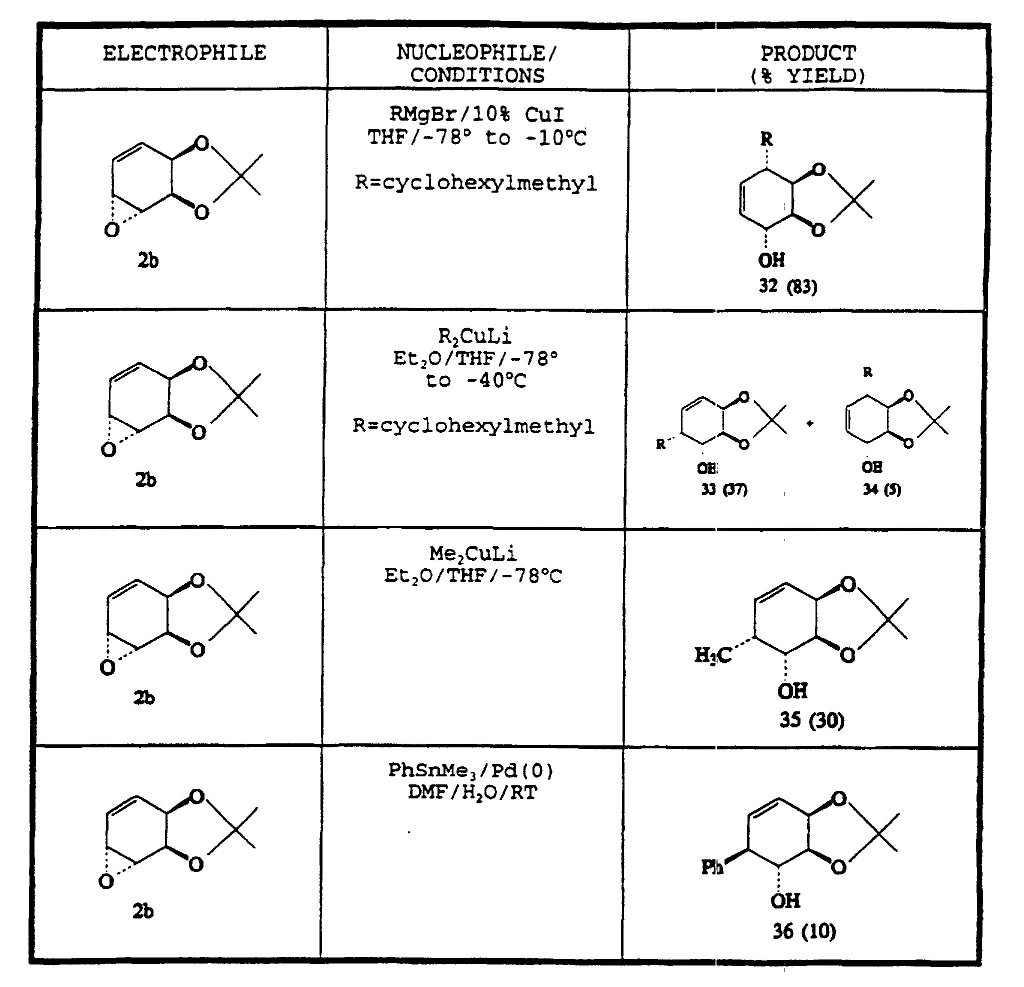

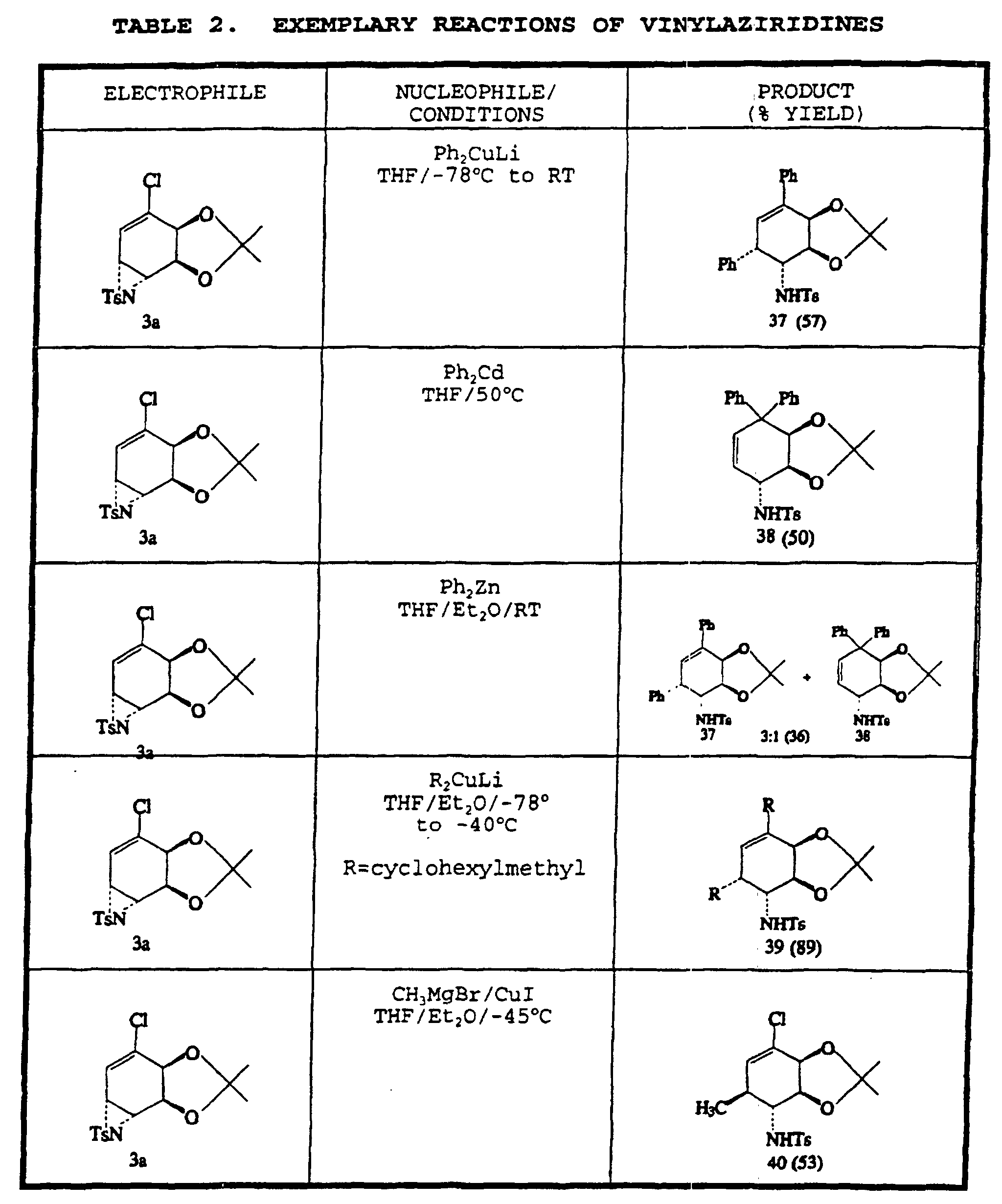

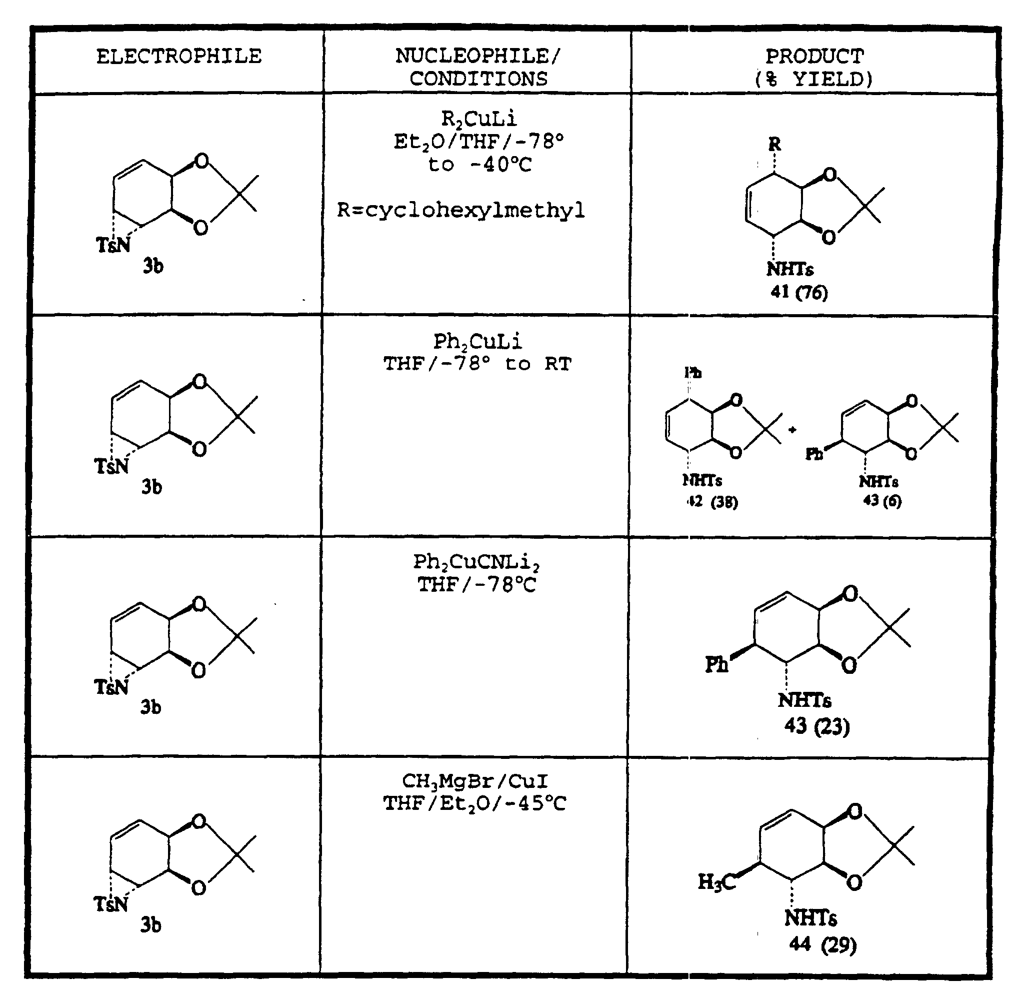

### EXPERIMENTAL

**General:** All reactions were carried out in an argon atmosphere with standard techniques for the exclusion of air and moisture. Glassware used for moisture sensitive reactions was flame dried under vacuum. Tetrahydrofurane, diethylether (ether) and toluene were distilled from Na benzophenone ketyl. ¹H NMR spectra were recorded at 270 MHz or 400 MHz, ¹³C NMR spectra at 50 MHz, 68 MHz or 100.6 MHz. Flash column chromatography was performed on Merck silica gel (grade 60, 230-400 mesh). Elemental analysis was performed by Atlantic Microlabs, Norcross, GA.

**General procedure for the formation of aziridines 3a and 3c:** A mixture of 5 equivalents (eq.) of (1*S*,2*S*)-3-halo-1,2-isopropylidenedioxycyclohexa-3,5-diene, 1 eq. of *p*-tosyliminophenyliodinane (PhI=NTs) and 0.08 eq. of copper acetyl acetonate (Cu(acac)₂) in 10 mL mmol⁻¹ of CH₃CN was stirred at room temperature (rt). After consumption of PhI=NTs the mixture was filtered through a pad of silica gel and concentrated *in vacuo.* The crude product was recrystallized from hexane/ethyl acetate.

### Example 1

**(1R,4S,5S,6R)-3-Chloro-4,5-isopropylidenedioxy-7-(4'-methyl-phenyl)sulfonylbicyclo[4.1.0]hept-2-ene (3a):** The compound was obtained in 20.5% yield from (1*S*,2*S*)-3-chloro-1,2-isopropylidenedioxycyclohexa-3,5-diene following the general procedure set forth above (reaction time 18 h): white solid; mp 202-203°C (hexane, ethyl acetate); [α]_{D}²⁵ -75.5° (c=1.54, CHCl₃) : ¹H NMR (270 MHz, CDCl₃) δ 7.82(dm, *J*=8.2 Hz, 2H), 7.37 (dm, *J*=8.2 Hz, 2H), 6.09 (dd, *J*=4.9, 1.2 Hz, 1H), 4.65 (ddd, *J*=6.6, 1.8, 0.7 Hz, 1H), 4.30 (dd, *J*=6.6, 1.0 Hz, 1H), 3.44 (dd, *J*=6.5, 1.8 Hz, 1H), 3.34 (dt, *J*=0.6, 6.5 Hz, 1H), 2.46 (s, 3H), 1.41 (s, 3H), 1.38 (s, 3H); ¹³C NMR (68 MHz, CDCl₃) δ 145.3 (C), 138.06 (C), 134.41 (C),130.06 (2CH), 128.07 (2CH), 119.96 (CH), 111.72 (C), 73.04 (CH), 71.68 (CH), 37.17 (CH), 36.74 (CH), 27.51 (CH₃), 26.07 (CH₃), 21.74 (CH₃); MS (CI+) *m*/*z* (rel. intensity) 356 (M+H⁺) (3), 340 (6), 298 (27), 262 (23), 200 (36), 155 (100), 142 (36), 114 (60), 91 (43). Anal. Calcd. for C₁₆H₁₈ClNO₄: C, 54.00; H, 5.12; N, 3.94. Found: C, 53.92; H, 5.12; N, 3.86.

### Example 2

**(1R,4R,5S,6R)-3-Bromo-4,5-isopropylidenedioxy-7-(4'-methylphenyl)-sulfonylbicyclo[4.1.0]hept-2-ene (3c):** From 10.52 g (45.52 mmol) of (1*S*,2*S*)-3-bromo-1,2-isopropylidenedioxycyclohexa-3,5-diene, 3c (1.97 g, 54% yield) was obtained following the general procedure described above (reaction time 1 h): white solid; mp 206-207°C (hexane, ethyl acetate); [α]_{D}²⁵ -33.7° (c=1.05 CHCl₃) ; ¹H NMR (270 MHz, CDCl₃) δ 7.82 (dm, *J*=8.2 Hz, 2H), 7.37 (dm, *J*=8.2 Hz, 2H), 6.35 (dd, *J*=4.9, 1.3 Hz, 1H), 4.64 (ddd, *J*=6.5, 1.7, 0.6 Hz, 1H), 4.34 (dd, *J*=6.5, 1.2 Hz, 1H), 3.44 (dd, *J*=6.5, 1.8 Hz, 1H), 3.28 (dd, *J*=6.5, 5.1 Hz, 1H), 2.46 (s, 3H), 1.42 (s, 3H), 1.38 (s, 3H) ; ¹³C NMR (100.6 MHz, CDCl₃) δ 145.1 (C), 134.1 (C), 129.92 (2CH), 129.89 (C), 127.9 (2CH), 123.9 (CH), 111.5 (C), 73.8 (CH), 71.4 (CH), 37.4 (CH), 36.4 (CH), 27.4 (CH₃), 26.1 (CH₃), 21.6 (CH₃); MS (CI+) *m*/*z* (rel. intensity) 400 (M+H⁺) (2), 384 (1.5), 372 (1.5), 344 (23), 314 (12), 262 (29), 244 (11), 228 (7), 187 (29), 155 (100), 108 (60), 91 (31); HRMS (Cl+) *m*/*z* calcd for (C₁₆H₁₈BrNO₄S+H) 400.0218, found 400.0231.

### Example 3

**1R,4R,5S,6R)-4,5-Isopropylidenedioxy-7-(4'-methylphenyl) sulfonylbicyclo[4.1.0]hept-2-ene (3b):** A mixture of 617 mg (1.54 mmol) of **3c**, 896 mg (3.09 mmol) of tributyltin hydride and 23 mg of AIBN in 25 mL of toluene was stirred under reflux. After 3 h, another 20 mg of AIBN was added and reflux was continued for 2.5 h. The mixture was washed with excess saturated KF aqueous solution, and the organic layer was dried over Na₂SO₄. Removal of solvent and column chromatography (silica gel, 3:1 hexane/EtOAc) afforded 288 mg (58%) of **3b**: white solid; ¹H NMR (270 MHz, CDCl₃) δ 7.82 (dm, *J*=8.2 Hz, 2H),7.35 (br.d, *J*=8.0 Hz, 2H), 5.95 (ddd, *J*=10.2, 4.4, 1.7 Hz, 1H), 5.76 (dd, *J*=10.2, 2.4 Hz, 1H), 4.54 (dd, *J*=6.7, 1.5 Hz, 1H), 4.39 (dt, *J*=6.7, 1.0 Hz, 1H), 3.37 (dd, *J*=6.5, 1.8 Hz, 1H), 3.27 (dd, *J*=6.5, 4.7 Hz, 1H), 2.46 (s, 3H), 1.37 (s, 3H), 1.34 (s, 3H); ¹³C NMR (68 MHz, CDCl₃) δ 144.8, 134.6, 132.4, 129.8 (2C), 127.9 (2C), 120.9 110.7, 70.6, 69.3, 36.4, 35.5, 27.8, 26.1, 21.6.

### Example 4

**(1R,2S,5S,6S)-4-Chloro-5,6-isopropylidenedioxy-2-methylcyclohex-3-en-1-ol (25):** 0.65 mL (1.95 mmol) of 3 M methylmagnesium bromide in ether was added to a suspension of 39 mg (0.20 mmol) of CuI in 7 mL of ether at -40°C. The mixture was stirred for 15 min before it was cooled to -78°C. A solution of 307 mg (1.52 mmol) of **2a** in 3 mL of ether was added and the mixture was stirred at -78°C for 2.5 h, then warmed slowly to -40°C. After 2 h, the reaction was quenched with 3 mL of saturated ammonium chloride solution, and the reaction mixture was extracted with ether (3x20 mL). The combined ether layers were dried over Na₂SO₄, and concentrated *in vacuo.* The residue was chromatographed on silica gel, eluted with 2:1 hexane/ethyl acetate to afford 293 mg (58%) of **25**: colorless oil; bp 100-110°C (0.1 mm, Kugelrohr); [α]_{D}²⁵ -21° (c=1.74, CHCl₃); IR (neat) 3460, 2990 2930, 2880, 1375, 1240, 1210, 1160, 1080, 1065, 1050, 925, 870 cm⁻¹; ¹H NMR (270 MHz, CDCl₃) δ 5.79 (d, *J*=2.0 Hz, 1H), 4.60 (dd, *J*=6.4, 1.4 Hz, 1H), 4.05 (dd, *J*=8.9, 6.3 Hz, 1H), 3.34 (dt, *J*=1.8, 9.0 Hz, 1H), 2.55 (d, *J*=2.3 Hz, 1H), 2.26 (m, 1H), 1.57 (s, 3H), 1.45 (s, 3H), 1.18 (d, *J*=7.1 Hz, 3H); ¹³C NMR (68 MHz, CDCl₃) δ 145.3 (C), 138.06 (C), 134.41 (C), 130.06 (2CH), 128.07 (2CH), 119.96 (CH), 111.72 (C), 73.04 (CH), 71.68 (CH), 37.17 (CH), 36.74 (CH), 27.51 (CH₃), 26.07 (CH₃), 21.74 (CH₃); MS (EI, 70 eV) m/z (rel. intensity) 203 (M⁺-CH₃) (100), 143 (87), 115 (81), 79 (54); HRMS (CI+) *m*/*z* calcd for (C₁₀H₁₅ClO₃ + H) 219.0788, found 219.0794.

### Example 5

**(1R,2S,5S,6S)-(4-Chloro-5,6-isopropylidenedioxy-2-cyclohexylmethylcyclohex-3-en-1-ol (26):** To a suspension of 364 mg (14.97 mmol) of Mg and a small crystal of iodine in 3 mL of THF a solution of 1.6 mL of cyclohexylmethyl bromide in 15 mL of THF was added over 1 h and the mixture was stirred at rt for 1.5 h. The resulting Grignard reagent was added into a suspension of 160 mg (0.84 mmol) of CuI in 4 mL of THF at -40°C. The mixture was stirred for 15 min, then cooled to -78°C, and a solution of 1.707 g (8.42 mmol) of **2a** in 8 mL of THF was added dropwise. The reaction mixture was warmed slowly to 0°C and stirred at 0°C for 3 h. The reaction was quenched with 5 mL of saturated NH₄Cl solution and the mixture was extracted with ethyl acetate (3x15 mL). The combined organic layers were dried over Na₂SO₄. Evaporation of the solvent and chromatography (hexane/EtOAc) afforded 995 mg (39%) of **26**.

¹H NMR (270 MHz, CDCl₃) δ 5.91 (d, *J*=2.10 Hz, 1H), 4.59 (dd, *J* =6.28,1.24 Hz, 1H), 4.05(dd, *J* =8.59, 6.30 Hz, 1H), 3.38 (dt, *J* =8.80, 2.84 Hz, 1H), 2.28 (bs, 1H), 2.24 (m, 1H), 1.53 (s, 3H), 1.42 (s, 3H), 0.70-1.90 (m, 13H). ¹³C NMR (68 MHz, CDCl₃) δ 131.41 (CH), 127.75 (C), 110.34 (C), 79.91 (CH), 75.89 (CH), 72.97 (CH), 38.64 (CH), 38.39 (CH₂), 34.73 (CH), 34.43 (CH₂), 32.50 (CH₂), 28.29 (CH₃), 26.56 (CH₂), 26.31 (CH₂), 26.12 (CH₂), 25.99 (CH₃).

### Example 6

**(1R,2R,5R,6S)-2,4-Di(cyclohexylmethyl)-5,6-isopropylidenedioxy-cyclohex-3-en-1-ol (27) :** To a suspension of 215 mg (31 mmol) of lithium in 12 mL of THF at -30°C was added 0.865 mL (6.2 mmol) of cyclohexylmethyl bromide at -30°C. The mixture was stirred 2 h before it was cannulated to a suspension of 590 mg (3.10 mmol) of cuprous iodide in 3 mL of ether precooled to -35°C. The mixture was stirred at -40°C for 40 min and cooled to -78°C, and a solution of 203 mg (1.00 mmol) of **2a** in 3 mL of THF was added. The resulting mixture was stirred at -78°C for 2 h and then allowed to warm up to -40°C before the reaction was quenched with 5 mL of saturated aqueous NH₄Cl solution. The aqueous layer was extracted with ethyl acetate (3x10 mL), and the combined organic phases were dried over Na₂SO₄. Removal of solvent and flash column chromatography (silica gel, hexane/ethyl acetate, 4:1) afforded 52 mg (14%) of **27** and 12 mg (4%) of **28**.

For 27, ¹H NMR (270 MHz, CDCl₃) δ 5.35 (d, *J* =3.55 Hz,1H), 4.47 (d, *J* =6.09 Hz, 1H), 4.24 (t, *J* =6.15 Hz, 1H), 3.90 (bs, 1H), 2.52 (bs, 1H), 2.19 (dd, *J* =13.96, 5.40 Hz, 1H), 1.41 (s, 3H), 1.39 (s, 3H). ¹³C NMR (68 MHz, CDCl₃) δ 134.32 (C), 127.94 (CH), 108.85 (C), 76.51 (CH), 73.96 (CH), 71.05 (CH), 41.99 (CH₂), 38.02 (CH₂), 35.29 (CH), 34.98 (CH), 34.99 (CH), 33.87 (CH₂), 33.25 (CH₂), 32.81 (CH₂), 27.73 (CH₃), 26.62 (2CH₂), 26.32 (2CH₂), 25.94 (CH₃).

### Example 7

**(1R,5R,6S)-5,6-Isopropylidenedioxy-4,4-diphenylcyclohex-2-en-1-ol (29):** To a suspension of 596 mg (3.12 mmol) of CuI in 5 mL of ether was added 3.5 mL of 1.8 M phenyllithium solution at 0°C. The mixture was stirred for 30 min and then a solution of 634 mg (3.13 mmol) of **2a** in 5 mL of ether was added. The mixture was stirred at 0°C for 2 h and at rt for 8 h before the reaction was quenched with 5 mL of ice water. The aqueous layer was extracted with ethyl acetate (3x15 mL), and the combined organic layers were dried over Na₂SO₄. Removal of solvent and column chromatography (silica gel, 2:1 hexane/ethyl acetate) gave 80 mg (8% yield) of **29.**

¹H NMR (270 MHz, CDCl₃) δ 7.10-7.40 (m, 10H), 6.38 (d, *J* =9.93 Hz, 1H), 6.11 (dd, *J* =9.93,3.59 Hz, 1H), 5.01 (d, *J* =6.75 Hz, 1H), 4.41 (dd, *J* =6.77,3.74 Hz, 1H), 4.29 (m, 1H), 1.93 (d, *J* =6.75 Hz, 1H), 1.36 (s, 3H), 1.27 (s, 3H). ¹³C NMR (68 MHz, CDCl₃) δ 147.77 (c), 143.43 (C), 136.12 (CH), 129.86 (CH), 129.49 (2CH), 128.56 (2CH), 127.69 (CH), 127.50 (2CH), 126.64 (CH), 126.20 (CH), 108,79 (C), 81.05 (CH), 79.90 (CH), 69.70 (CH), 52.39 (C), 26.62 (CH₃), 25.14 (CH₃).

### Example 8

**(1R,2S,5R,6S)-5,6-Isopropylidenedioxy-4-methylcyclohex-3-en-1-ol (30) and (1R,2S,5R,6S)-5,6-Isopropylidenedioxy-2-methylcyclohex-3-en-1-ol (31):** To a suspension of CuI (29 mg, 0.15 mmol) in anhydrous ether was added a solution of methylmagnesium bromide (MeMgBr) (0.515 mL, 1.54 mmol) at -40°C and stirred for 30 min. At -78°C compound **2b** (200 mg, 1.19 mmol) in anhydrous THF (5 mL) was added precooled via cannula and the mixture was warmed slowly to - 40°C and stirred for 2 h at -40°C. MeMgBr (0.25 mL, 0.75 mmol) was added. After 30 min the white suspension was quenched using saturated NH₄Cl solution (20 mL) and the mixture was extracted with CH₂Cl₂ (4x). The combined organic phases were washed with brine, dried over Na₂SO₄ and concentrated *in vacuo.* Chromatography (toluene/ethyl acetate, 75:25) afforded **30** (77 mg, 35%) and **31** (24 mg, 11%).

**30:** colorless oil; ¹H NMR (270 MHz, CDCl₃) δ 5.75 (ddd, *J*=9.7, 3.0, 2.2 Hz, 1H), 5.50 (dt, *J*=9.6, 2.9 Hz, 1H), 4.20 (m, 1H), 4.00 (dd, *J*=7.8, 6.2 Hz, 1H), 3.81 (t, *J*=7.3 Hz, 1H), 2.76 (br.s, 1H), 21.7 (m, 1H), 1.48 (s, 3H), 1.36 (s, 3H), 1.24 (d, *J*=7.2, 1H); ¹³C NMR (68 MHz, CDCl₃) δ 131.89, 130.74, 108.88, 81.44, 79.35, 71.90, 35.12, 27.26, 24.75, 18.91.

**31**: colorless oil; ¹H NMR (270 MHz, CDCl₃) δ 5.80 (ddd, *J*=9.8, 3.5, 2.8 Hz, 1H), 5.65 (br.d, *J*=9.8 Hz, 1H), 4.57 (m, 1H), 3.96 (dd, *J*=9.0, 6.3 Hz, 1H), 3.27 (dt, *J*=2.6, 9.3 Hz, 1H), 2.73 (d, *J*=2.4 Hz, 1H), 2.13 (m, 1H), 1.48 (s, 3H), 1.37 (s, 3H), 1.14 (d, *J*=7.1, 1H); ¹³C NMR (68 MHz, CDCl₃) δ 136.8, 122.4, 109.4, 79.7, 75.3, 72.8, 35.6, 28.4, 25.8, 17.1.

### Example 9

**(1R,4S,5R,6S)-4-Cyclohexylmethyl-5,6-isopropylidenedioxycyclohex-2-en-1-ol (32):** To a suspension of 39 mg (0.20 mmol) of cuprous iodide in 3 mL of THF cooled to -40°C was added 2.69 mmol of cyclohexylmethyl magnesium bromide. The mixture was stirred at - 40°C for 15 min then cooled to -78°C, when solution of 334 mg (1.99 mmol) of **2b** in 4 mL of THF was added. The mixture was warmed to - 10°C with stirring before the reaction was quenched with 5 mL of saturated NH₄Cl solution. The aqueous layer was extracted with ethyl acetate (3x15 mL), and the combined organic layers were dried over Na₂SO₄. The product was purified using column chromatography (silica gel, hexane/ethyl acetate, 3:1) to give 439 mg (83%) of **32.**

¹H NMR (270 MHz, CDCl₃) δ 5.76 (dt, *J* =9.71,2.57 Hz, 1H), 5.61 (dt, *J* =9.71, 2.77 Hz, 1H), 4.21 (m, 1H), 3.97 (dd, *J* =7.66,6.08 Hz, 1H), 3.83 (dd, *J* =7.51, 6.56 Hz, 1H), 2.58 (bs, 1H), 2.19 (m, 1H), 1.47 (s, 3H), 1.36 (s, 3H). ¹³C NMR (68 MHz, CDCl₃) δ 130.74 (CH), 130.54 (CH), 108.67 (C), 81.40 (CH), 78.30 (CH), 71.61 (CH), 41.42 (CH₂), 37.40 (CH), 35.10 (CH), 33.94 (CH₂), 32.81 (CH₂), 27.30 (CH₃), 26.56 (CH₂), 26.25 (CH₂), 26.12 (CH₂), 24.88 (CH₃).

### Example 10

**(1R, 2R,5R,6S)-2-Cyclohexylmethyl-5,6-isopropylidenedioxycyclohex-3-en-1-ol (33):** To a suspension of 350 mg (50.4 mmol) of lithium in 17 mL of ether at -30°C was added 1.395 mL (10.0 mmol) of cyclohexylmethyl bromide at -30°C. The mixture was stirred at - 30°C for 2 h before it was cannulated to a suspension of 952 mg (5.00 mmol) of cuprous iodide in 5 mL of ether precooled to - 40°C. The mixture was stirred at -40°C for 40 min and cooled to - 78°C, when a solution of 279 mg (1.66 mmol) of **2b** in 4 mL of THF was added. The resulting mixture was stirred at -78°C for 2 h before the reaction was quenched by 5 mL of saturated aqueous NH₄Cl solution. The aqueous layer was extracted with ethyl acetate (3x15 mL), and the combined organic phases were dried over Na₂SO₄. Removal of solvent and column chromatography (silica gel, CH₂Cl₂/acetone, 5:1) afforded 165 mg (37%) of **33** and 25 mg (5%) of **32**.

### Example 11

**(1R,2R,5R,6S)-5,6-Isopropylidenedioxy-2-methylcyclohex-3-en-1-ol (35):** At -30°C methyllithium (2.55 mL, 3.57 mmol) was added slowly to a stirred suspension of cuprous iodide (340 mg, 1.78 mmol). After 30 min compound **2b** (110 mg, 0.65 mmol) was added precooled by cannula at -78°C. The reaction was quenched after 30 min with saturated NH₄Cl solution (10 mL). The mixture was extracted with CH₂Cl₂ (4x). The combined extracts were washed with brine, dried over MgSO₄ and concentrated *in vacuo.* Chromatography (hexane/ethyl acetate, 67:33) afforded **35** (36 mg, 30%): colorless oil; ¹H NMR (270 MHz, CDCl₃) δ 5.85 (dd, *J*=10.0, 4.3 Hz, 1H), 5.77 (ddd, *J*=10.0, 3.4 Hz, 1H), 4.63 (dd, *J*=6.1, 3.4 Hz, 1H), 4.20 (dd, *J*=7.5, 6.2 Hz, 1H), 3.87 (dd, *J*=7.5, 4.7 Hz, 1H), 2.55 (m, 1H), 1.80 (s, 1H), 1.47 (s, 3H), 1.40 (s, 3H), 1.07 (d, *J*=7.3, 1H); ¹³C NMR (100.6 MHz, CDCl₃) δ 135.13, 123.38, 109.03, 76.27, 72.12, 71.61, 33.30, 28.12, 25.87, 14.41.

### Example 12

**(1R,2R,5R,6S)-5,6-Isopropylidenedioxy-2-phenylcyclohex-3-en-1-ol (36):** To a degassed solution of compound **2b** (200 mg, 1.19 mmol) in DMF (2.0 mL) and H₂O (0.20 mL, 11 mmol) Pd(CH₃CN)₂Cl₂ (15 mg, 0.058 mmol) was added under argon. After 5 min phenyltrimethyltin (344 mg, 1.43 mmol) was added to the orange solution which decolored to a pale yellow. The initially exothermic reaction was cooled to maintain rt. After 10 min more phenyltrimethyltin (160 mg) was added. After complete consumption of **2b** (20 min) the black mixture was diluted (CH₂Cl₂, 30 mL), filtered over Celite®, washed (H₂O, brine), dried over Na₂SO₄, and concentrated *in vacuo*. Chromatography (hexane/ethyl acetate, 67:33) afforded **36** (29 mg, 10%): white solid; mp. 95-96°C; IR (KBr) 3490, 3090, 3040, 3000, 2940, 2880, 1495, 1455, 1382, 1370, 1250, 1160, 1070, 1055, 903, 8430, 860, 800, 755, 705 cm⁻¹; ¹H NMR (400 MHz, CDCl₃) δ 7.36 (t, *J*=7.2 Hz, 2H), 7.29 (t, *J*=7.3 Hz, 1H), 7.25 (d, *J*=7.2 Hz, 2H), 603 (ddd, *J*=9.9, 3.7, 3.0 Hz, 1H), 5.88 (dt, *J*=9.9, 1.2 Hz, 1H), 4.73 (m, 1H), 4.17 (dd, *J*=8.8, 6.4 Hz, 1H), 3.62 (dt, *J*=1.2, 9.3 Hz, 1H), 3.28 (ddt, *J*=9.8, 2.8, 1.5 Hz, 1H), 2.06 (d, *J*=2.0 Hz, 1H), 1.53 (s, 3H), 1.42 (s, 3H); ¹³C NMR (100.6 MHz, CDCl₃) δ 140.82, 134.61, 128.78 (2C), 128.38 (2C) 127.29, 124.17, 109.75, 79.02, 75.13, 72.74, 48.22, 28.33, 25.77; MS (Cl+) *m*/*z* (rel. intensity) 231 (M⁺-15) (100), 171 (72), 159 (43), 143 (71), 128 (30), 115 (30), 101 (39), 91 (54).

### Example 13

**(1R,2S,5R,6S)-*N*-(5,6-Isopropylidenedioxy-2,4-diphenylcyclohex-3-enyl)-(4'-methylphenyl)sulfonamide (37):** Phenyllithium (0.47 mL, 0.84 mmol) was added to a suspension of CuI (80 mg, 0.42 mmol) in anhydrous THF at -40°C and stirred for 15 min. Compound **3a** (50 mg, 0.14 mmol) in anhydrous THF (2 mL) was added precooled by cannula, followed by BF₃Et₂O (60 mg, 0.42 mmol), and the mixture was stirred and allowed to warm to rt over 12 h. The reaction was quenched using NH₄OH solution, solid NH₄Cl was added and the mixture was extracted with ether (5x). The combined extracts were washed with brine, dried over MgSO₄ and concentrated *in vacuo*. Chromatography afforded **37** (35 mg, 52%): white solid; mp. 268-270°C: [α]_{D}²⁵ -105.2° (c=0.50, CHCl₃); ¹H NMR (270 MHz, CDCl₃) δ 7.59 (m, 4H), 7.08-7.41 (m, 10H), 6.34 (d, *J*=4.8 Hz, 1H), 5.19 (d, *J*=5.7 Hz, 1H), 4.37 (d, *J*=6.6 Hz, 1H), 4.27 (dd, *J*=8.3, 5.5 Hz, 1H), 4.14 (t, *J*=5.1 Hz, 1H), 3.62 (ddd, *J*=8.1, 6.6, 5.2 Hz, 1H), 2.40 (s, 3H), 1.38 (s, 3H), 1.14 (s, 3H); ¹³C NMR (100.6 MHz, CDCl₃) δ 143.19 (C), 138.66 (C), 136.96 (C), 136.45 (C), 136.26 (C), 129.62 (2CH), 129.51 (2CH), 128.63 (2CH), 128.61 (2CH), 128.00 (CH), 127.35 (4CH), 125.97 (2CH), 109.56 (C), 74.21 (CH), 72.98 (CH), 55.26 (CH), 43.92 (CH), 27.28 (CH₃), 25.95 (CH₃), 21.45 (CH₃) ; MS (CI+) *m*/*z* (rel. intensity) (M+H⁺ not found), 418 (7), 400 (18), 247 (82), 222 (87), 139 (30), 98 (68), 91 (100).

### Example 14

**(1R,5R,6S)-*N*-(4,4-Diphenyl-5,6-isopropylidenedioxycyclohex-2-enyl)-(4'-methylphenyl)sulfonamide (38):** To a suspension of anhydrous CdCl₂ (103 mg, 0.56 mmol) in anhydrous THF (10 mL) phenyllithium (0.62 mL, 1.12 mmol) was added slowly at rt, and the yellow solution was heated at reflux for 45 min. After cooling to rt compound **3a** (100 mg, 0.28 mmol) in anhydrous THF (5 mL) was added and the mixture was heated to 55°C for 27 h. Saturated NH₄Cl solution (15 mL) was added and the mixture was extracted with ether (4x). Drying over MgSO₄, concentration *in vacuo* and chromatography (hexane/ethyl acetate, 80:20) afforded **38** (67 mg, 50%); glassy solid; ¹H NMR (400 MHz, CDCl₃) δ 7.65 (dm, *J*=8.4 Hz, 2H), 7.14-7.39 (m, 12H), 6.53 (dt, *J*=9.9, 1.1 Hz, 1H), 5.82 (ddd, *J*=10.0, 5.4, 0.6 Hz, 1H), 5.07 (dd, *J*=6.1, 1.4 Hz, 1H), 4.37 (dd, *J*=6.1, 1.4 Hz, 1H), 3.87 (dd, *J*=9.3,5.3 Hz, 1H), 3.81 (d, *J*=9.3 Hz, 1H), 2.42 (s, 3H), 1.256 (s, 3H), 1.249 (s, 3H); ¹³C NMR (100.6 MHz, CDCl₃) δ 146.73 (C), 144.85 (C), 143.57 (C), 138.45 (CH), 138.03 (C), 129.82 (2CH), 129.20 (2CH), 129.02 (2CH), 127.93 (2CH), 127.80 (2CH), 127.55 (CH), 127.34 (2CH), 126.59 (CH), 126.40 (CH), 108.67 (C), 80.04 (CH), 78.57 (CH), 52.96 (CH), 50.80 (C), 26.94 (CH₃), 25.22 (CH₃), 21.76 (CH₃); MS (CI+) *m*/*z* (rel. intensity) (M+H⁺ not found), 418 (14), 400 (12), 388 (10), 375 (12), 276 (22), 247 (100), 219 (45), 172 (23), 155 (28), 91 (100).

### Example 15

**(1R,2R,5R,6S)-*N*-[2,4-Di(cyclohexylmethyl)-5,6-isopropylidene-dioxycyclohex-3-enyl]-(4'-methylphenyl)sulfonamide (39):** To a suspension of 193 mg (27.8 mmol) of lithium in 12 mL of ether cooled to -30°C was added 0.766 mL (5.56 mmol) of cyclohexylmethyl bromide. The mixture was stirred at -30°C for 2 h and cannulated to a suspension of 529 mg (2.78 mmol) of cuprous iodide in 3 mL of ether precooled to -40°C. After the mixture was stirred at -40°C for 40 min, it was cooled to -78°C and a solution of 329 mg (0.92 mmol) of **3a** in 5 mL of THF was added. The mixture was stirred at -78°C for 2 h, then slowly warmed to -40°C and stirred at -40'C for 2 h, then quenched with 5 mL of saturated aqueous ammonium chloride and extracted with ethyl acetate (3x5 mL). The combined organic layers were dried over Na₂SO₄ and concentrated *in vacuo.* The residue was chromatographed (silica gel, 4:1 hexane/EtOAc) to give 433 mg (89%) of **39.**

White solid, mp 192-193°C; ¹H NMR (270 MHz, CDCl₃) δ 7.76 (d, *J* =8.30 Hz, 2H), 7.29 (d, *J* =8.09, 2H), 5.29 (bs, 1H), 4.46 (d, *J* =6.28 Hz, 1H), 4.40 (d, *J* =5.83 Hz, 1H), 4.28 (t, *J* =5.97 Hz, 1H), 3.38 (q, *J* =4.51 Hz, 1H), 2.65 (bs, 1H), 2.41 (s, 3H), 2.11 (dd, *J* =14.26, 5.52 Hz, 1H), 1.82 (dd, *J* =14.15, 8.56 Hz, 1H), 1.31 (s, 3H), 1.14 (s, 3H), 0.50~1.72 (m, 24H). ¹³C NMR (68 MHz, CDCl₃) δ 143.43 (C), 137.54 (C), 135.44 (C), 129.65 (2CH), 128.19 (CH), 127.39 (2CH), 109.28 (C), 75.01 (CH), 73.59 (CH), 55.06 (CH), 42.10 (CH₂), 38.20 (CH₂), 35.29 (CH), 34.55 (CH), 33.87 (CH₂), 33.62 (CH₂), 33.02 (2CH₂), 32.06 (CH), 27.30 (CH₃), 26.62 (2CH₂), 26.26 (2CH₂), 26.06 (CH₂), 25.93 (CH₃), 21.41 (CH₃). Anal. Calcd for C₃₀H₄₅O₄NS:C, 69.86; H, 8.79; N, 2.72. Found: C, 69.92; H, 8.81; N, 2.69.

### Example 16

**(1R,2S,5S,6S)-*N*-(4-Chloro-5,6-isopropylidenedioxy-2-methylcyclohex-3-enyl)-(4'-methylphenyl)sulfonamide (40):** To a suspension of CuI (14 mg, 0.073 mmol) in anhydrous ether was added a solution of methylmagnesium bromide (MeMgBr) (0.050 mL, 0.15 mmol) at -45°C and stirred for 30 min. Compound **3a** (200 mg, 0.56 mmol) in anhydrous THF (5 mL) was added precooled via cannula and MeMgBr (0.40 mL, 1.20 mmol) was added over 60 min. After 7 h the white suspension was quenched using saturated NH₄Cl solution (containing NH₃, pH 9) (30 mL) and the mixture was extracted with ether (4x). The combined organic phases were washed with brine, dried over Na₂SO₄ and concentrated *in vacuo*. Chromatography (toluene/ethyl acetate, 80:20) afforded **40** (111 mg, 53%): ¹H NMR (400 MHz, CDCl₃) δ 7.79 (dm, *J*=8.2 Hz, 2H), 7.29 (dm, *J*=8.7 Hz, 2H), 5.79 (d, *J*=3.0 Hz, 1H), 5.02 (d, *J*=8.5 Hz, 1H), 4.50 (dd, *J*=6.0, 1.4 Hz, 1H), 4.08 (dd, *J*=7.9, 6.0 Hz, 1H), 3.28 (q, *J*=8.0 Hz, 1H), 2.45 (s, 1H), 2.19 (ddquintett, *J*=1.4, 3.1, 7.3 Hz, 1H), 1.282 (s, 3H), 1.258 (s, 3H), 1.13 (d, *J*=7.2 Hz, 3H); ¹³C NMR (100.6 MHz, CDCl₃) δ 143.2, 138.3, 132.1, 129.3 (2C), 128.4, 127.2 (2C), 110.3, 77.7, 75.2, 57.2, 36.5, 27.4, 25.8, 21.5, 18.0.

### Example 17

**(1R,4S,5R,6S)-*N*-(4-Cyclohexylmethyl-5,6-isopropylidene-dioxycyclohex-2-enyl)-(4'-methylphenyl)sulfonamide (41):** To a suspension of 187 mg (26.94 mmol) of lithium in 12 mL of ether at - 30°C was added 0.726 mL (5.20 mmol) of cyclohexylmethyl bromide at - 30°C. The mixture was stirred at -30°C for 2 h before it was cannulated to a suspension of 495 mg (2.60 mmol) of cuprous iodide in 3 mL of ether precooled to -40°C. The mixture was stirred at - 40°C for 40 min and cooled to -78°C, when a solution of 281 mg (0.87 mmol) of **3b** in 4 mL of THF was added. The resulting mixture was stirred at -78°C for 2 h and then allowed to warm to -40°C before the reaction was quenched with 5 mL of saturated aqueous NH₄Cl solution. The aqueous layer was extracted with ethyl acetate (3x15 mL), and the combined organic phases were dried over Na₂SO₄. Removal of solvent and column chromatography (silica gel, hexane/ethyl acetate, 3:1) afforded 276 mg (76%) of **41.**

White solid; mp 138-139°C; [α]_{D}²⁰=40.4° (c=0.96, CHCl₃); ¹H NMR (270 MHz, CDCl₃) δ 7.79 (d, *J* =8.25 Hz, 2H), 7.30 (d, *J* =8.21 Hz, 2H), 5.66 (m, 2H), 4.70 (d, *J* =5.58 Hz, 1H), 3.81 (m, 2H), 3.58 (m, 1H), 2.42 (s, 3H), 2.22 (bs, 1H), 1.23 (s, 3H), 1.16 (s, 3H), 0.71-1.80 (m, 13H). ¹³C NMR (68 MHz, CDCl₃) δ 143.30 (C), 137.48 (C), 132.34 (CH), 132.34 (CH), 129.49 (CH), 128.00 (CH), 127.51 (CH), 108.79 (C), 78.36 (CH), 78.18 (CH), 54.81 (CH), 41.74 (CH₂), 37.03 (CH), 35.10 (CH), 33.80 (CH₂), 32.99 (CH₂), 27.24 (CH₃), 26.55 (CH₂), 26.23 (CH₂), 25.19 (CH₃), 21.30 (CH₃).

### Example 18

### (1R,4R,5R,6S)-N-(5,6-Isopropylidenedioxy-4-phenylcyclohex-2-enyl)-(4'-methylphenyl)sulfonamide (42) and (1R,2R,5R,6S)-N-(5,6-Isopropylidenedioxy-2-phenylcyclohex-3-enyl)-(4'-methylphenyl) sulfon-amide (43)

Method A: With lithium diphenylcuprate: 2.36 mL of 1.8 M phenyllithium solution was added slowly at -35°C to a suspension of 224 mg (1.18 mmol) of cuprous iodide in 8 mL of THF. The resulting mixture was stirred for 30 min, and a solution of 125 mg (0.39 mmol) of **3b** in 2 mL of THF was added followed by 0.145 mL of BF₃Et₂O. The mixture was warmed over 5 h to rt with stirring, quenched with 5 mL of saturated aqueous ammonium chloride solution and extracted with ethyl acetate (3x10 mL). The combined organic phases were dried over Na₂SO₄, removal of solvent and column chromatography afforded 54 mg (38%) of **42** and 10 mg (6%) of **43:** white solid; mp. 165-167°C; ¹H NMR (270 MHz, CDCl₃) δ 7.42 (dm, J=8.3 Hz, 2H), 7.26 (dm, *J*=8.3 Hz, 2H), 7.23 (m, 1H), 7.09 (m, 4H), 6.00 (ddd, *J*=9.9, 3.5, 2.7 Hz, 1H), 5.87 (dt, *J*=9.9, 1.5 Hz, 1H), 4.67 (brt, *J*=4.7 Hz, 1H), 4.52 (d, *J*=8.2 Hz, 1H), 4.14 (dd, *J*=9.0, 6.0 Hz, 1H), 3.65 (q, *J*=9.0 Hz, 1H), 3.24 (dq, *J*=8.6, 1.9 Hz, 1H), 2.38 (s, 3H), 1.44 (s, 3H), 1.33 (s, 3H); ¹³C NMR (100.6 MHz, CDCl₃) δ 142.4 (C), 140.1 (C), 138.6 (C), 134.6 (CH), 129.1 (2CH), 128.61 (2CH), 128.57 (2CH), 127.14 (CH), 126.96 (2CH), 124.2 (CH), 109.93 (C), 77.58 (CH), 72.16 (CH), 58.95 (CH), 47.70 (CH), 27.79 (CH₃), 25.83 (CH₃), 21.41 (CH₃).

Method B: **43** with dilithium diphenylcyanocuprate: a suspension of 161 mg (1.80 mmol) of dry CuCN in 2 mL of THF was treated with 2.0 mL of 1.8 M phenyllithium solution at -78°C. The mixture was warmed to -10°C with stirring to dissolve CuCN, and then cooled to -78°C, when a solution of 182 mg (0.57 mmol) of **3b** was added, followed by 0.221 mL of BF₃Et₂O. The mixture was stirred at -78°C for 3 h and then quenched with 5 mL of aqueous NH₄Cl solution (containing NH₃, pH 8). After stirring at rt for 30 min, the mixture was extracted with ethyl acetate (3x15 mL), the combined organic layers were dried over Na₂SO₄ and concentrated *in vacuo*. The residue was chromatographed on silica gel eluting with 10:1 CHCl₃/acetone to give 52 mg (23%) of **43.**

### Example 19

**43 and (1R,2R)-*N*-(6-Hydroxycyclohexa-2,4-dienyl)-(4'-methylphenyl)sulfonamide (24)** with phenyltrimethyltin/Pd(0)-catalysis: To a degassed solution of compound **3b** (80 mg, 0.249 mmol) in DMF (0.8 mL)/H₂O (0.045 mL, 2.5 mmol, 10 eq.) Pd(CH₃CN)₂Cl₂ (3.2 mg, 0.012 mmol) was added under argon. After 5 min phenyltrimethyltin (72 mg, 0.30 mmol) was added to the orange solution which decolored to a pale yellow. After 22 h and 37 h phenyltrimethyltin (2x72 mg) was added. When no more 3b remained the reaction mixture became black. After addition of 5 mL of water the mixture was extracted with ether (6x), dried over Na₂SO₄, and concentrated. Chromatography (toluene/ethyl acetate, 80:20) afforded **43** (18.6 mg, 19%) and **24** (6.6 mg, 10%): colorless oil; ¹H NMR (270 MHz, CDCl₃) δ 7.78 (dm, *J*=8.3 Hz, 2H), 7.31 (dm, *J*=8.0 Hz, 2H), 5.85 (m, 3H), 5.43 (m, 1H), 5.34 (br.d, *J*=8.3 Hz, 1H), 4.42 (br.d, *J*=10.6 Hz, 1H), 4.00 (ddm, *J*=10.6, 8.3 Hz, 1H), 2.90 (br.s, 1H), 2.40 (s, 3H); ¹³C NMR (68 MHz, CDCl₃) δ 143.8 (C), 137.2 (C), 129.8 (2CH), 129.6 (CH), 127.2 (2CH), 126.5 (CH), 125.6 (CH), 124.0 (CH), 71.2 (CH), 57.1 (CH), 21.5 (CH₃).

### Example 20

**(1R,2S,5R,6S)-*N*-(5,6-Isopropylidenedioxy-2-methylcyclohex-3-enyl)-(4'-methylphenyl)sulfonamide (44) :** To a suspension of CuI (7 mg, 0.035 mmol) in anhydrous ether a solution of methylmagnesium bromide (MeMgBr) (0.025 mL, 0.075 mmol) was added at -45°C and stirred for 30 min. Compound **3b** (113 mg, 0.35 mmol) in anhydrous THF (5 mL) was added precooled via cannula and MeMgBr (0.175 mL, 0.525 mmol) was added over 60 min. After 120 min saturated NH₄Cl solution (containing NH₃, pH 9) (30 mL) was added to the white suspension and the mixture was extracted with ether (4x). The combined organic phases were washed with brine, dried over Na₂SO₄ and concentrated in vacuo. Chromatography (hexane/ethyl acetate, 80:20) afforded **44** (34 mg, 29%): white solid; mp. 112-113°C; [α]_{D}²⁰ -54.0° (c=0.58, CHCl₃): 1R (CHCl₃) 3260, 2980, 2930, 1450, 1375, 1320, 1150, 1080, 1060, 860, 805 cm⁻¹; ¹H NMR (270 MHz, CDCl₃) δ 7.80 (dm, *J*=6.6 Hz, 2H), 7.28 (dm, *J*=7.9 Hz, 2H), 5.79 (ddd, *J*=10.0, 3.4, 2.4 Hz, 1H), 5.68 (ddd, *J*=10.9, 1.9, 0.8 Hz, 1H), 4.66 (d, *J*=8.7 Hz, 1H), 4.52 (m, 1H), 3.92 (dd, *J*=8.0, 6.2 Hz, 1H), 3.17 (q, *J*=9.0 Hz, 1H), 2.41 (s, 3H), 2.07 (m, 1H), 1.24 (s, 3H), 1.17 (s, 3H), 1.14 (d, *J*=7.2 Hz, 3H); ¹³C NMR (100.6 MHz, CDCl₃) δ 142.8 (C), 138.8 (C), 136.2 (CH), 129.2 (2CH), 127.3 (2CH), 122.9 (CH), 109.3 (C), 77.5 (CH), 72.2 (CH), 58.8 (CH), 35.75 (CH), 27.5 (CH₃), 25.7 (CH₃), 21.4 (CH₃), 18.0 (CH₃); MS (EI) *m*/*z* (rel. intensity) 337 (M⁺) (1.5), 322 (6), 254 (45), 125 (98), 91 (100).

## Claims

1. A method for preparing a desired compound which is a conjugate of units selected from cyclitols, carbohydrates and C-analogs thereof, the method comprising:
coupling an epoxide of the formula: 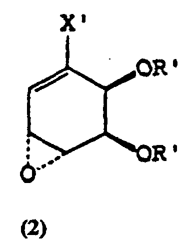
wherein:
X' is H, halogen, CN, alkyl, aryl, or a heteroatom; and each R' is independently any suitable alcohol protecting group, provided that the R' at alcohol C2 and C3 may be the same or different;
or an aziridine of the formula: 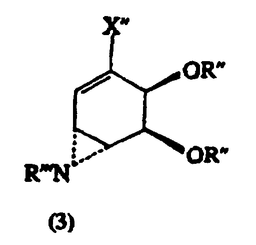 wherein:
X" is H, halogen, CN, alkyl, aryl, or a heteroatom; each R" is independently any alcohol protecting group, provided that the R" at alcohols C2 and C3 may be the same or different; and
R"' is H, CBZ, tosyl or any substituted or unsubstituted arylsulfonic acid amide, benzyl or CO₂Me;
under suitable conditions, with a nucleophilic cyclitol, sugar or C-sugar having a nucleophilic carbon atom or heteroatom, resulting in the coupling of the epoxide or aziridine with the cyclitol, sugar or C-sugar via a ring opening reaction, thereby generating a nucleophilic group which is available for a further reaction.

2. The method of claim 1, further comprising the step of employing the generated nucleophilic group in a further reaction with a further epoxide **(2)** or aziridine **(3)**.

3. The method of claim 1, wherein the desired compound is of the formula (1): 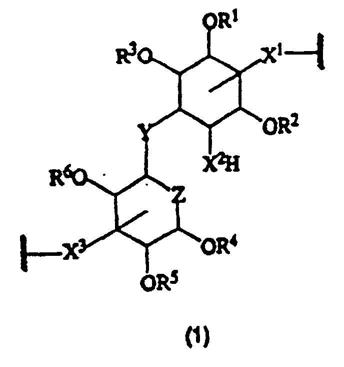 wherein:
X¹ is O, X² is O or NR''', and X³ is O, NH, S, or CH₂ ;
Y is O, CH₂, S, or NH;
Z is O, CH₂, NH or S; and
R¹ - R⁶ independently are H or any suitable alcohol protecting group;
the method comprising:
a) coupling an epoxide of the formula: 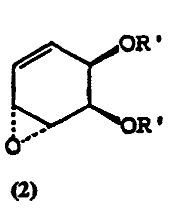 wherein:
each R' is independently any suitable alcohol protecting group, provided that the R' at alcohol C2 and C3 may be the same or different;
or an aziridine of the formula: 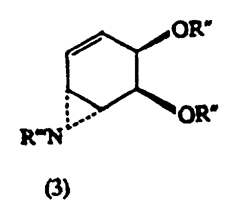 wherein:
each R" is independently any alcohol protecting group, provided that the R" at alcohols C2 and C3 may be the same or different; and R"' is H, CBZ, tosyl or any substituted or unsubstituted arylsulfonic acid amide, benzyl or CO₂Me;
under suitable conditions, with a nucleophilic cyclitol, sugar or C-sugar having a nucleophilic carbon atom or heteroatom, resulting in the coupling of the electrophile with the cyclitol or C-sugar by a ring opening reaction of said electrophile, thereby generating a nucleophilic group which is available for a further reaction; and further comprising
(b) converting the double bond present in the electrophilic group to the diol or protected diol (comprising OR¹ and OR³) of structure (**1**), and
(c) optionally deprotecting the compound of step (b).

4. The method of claim 3, further comprising the step of employing the generated nucleophilic group in a further reaction with a further epoxide **(2)** or aziridine **(3)**.

5. The method of claim 3, wherein the nucleophilic cyclitol, sugar or C-sugar is a heteroatom or carbon atom nucleophile comprising the portion of formula **(1)** shown bellow: 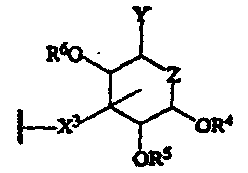 where X³, Y, Z, and R⁴-R⁶ are as defined above.

6. A method for preparing conduritol derivatives, comprising coupling an electrophile of formula (2), wherein X' is H, Br, or Cl, with a nucleophile comprising an organometallic reagent of the formula RMgBr, R₂CuLi, R₂CuCnLi₂, or RSnMe₃/Pd(0), wherein R is methyl, methylcyclohexyl or phenyl, under suitable conditions.

7. A method for preparing conduritol derivatives, comprising coupling an electrophile of formula (3), wherein X" is H, Br, or Cl, with a nucleophile comprising an organometallic reagent of the formula, RMgBr, R₂CuLi, R₂CuCnLi₂, or RSnMe₃/Pd(0), wherein R is methyl, methylcyclohexyl or phenyl, under suitable conditions.

8. A method of claim 1 or claim 3, wherein the desired compound is selected from the group consisting of a cyclitol conjugate, a carbohydrate conjugate, and a semi- or fully carbocyclic analog thereof.

9. A method of claim 1 or claim 3, wherein the desired compound is GM3 (54), silyl Lewis X (56), or a carbon or heteroatom conjugate of either.

10. A method of claim 6 or 7, wherein the conduritol derivative is selected from the group consisting of:
(1R,2R,5S,6S)-(4-Chloro-5,6-isopropylidenedioxy-2-methylcyclohex-3-en-1-ol **(25);**
(1R,2R,5S,6S)-(4-Chloro-5,6-isopropylidenedioxy-2-cyclohexylmethylcyclohex-3-en-1-ol **(26);**
(1R,2R,5R,6S)-2,4-Di(cyclohexylmethyl)-5,6-isopropylidenedioxy-cyclohex-3-en-1-ol **(27);**
(1R,5R,6S)-5,6-Isopropylidenedioxy-4,4-diphenylcyclohex-2-en-1-ol **(29);**
(1R,2S,5R,6S)-5,6-Isopropylidenedioxy-4-methylcyclohex-3-en-1-ol **(30)**;
(1R,2S,5R,6S)-5,6-Isopropylidenedioxy-2-methylcyclohex-3-en-1-ol **(31)**;
(1R,4S,SR,6S)-4-Cyclohexylmethyl-5,6-isopropylidenedioxycyclohex-2-en-1-ol **(32)**;
(1R,2R,5R,6S)-2-Cyclohexylmethyl-5,6-isopropylidenedioxycyclohex-3-en-1-ol **(33)**;
(1R,2R,5R,6S)-5,6-Isopropylidenedioxy-2-methylcyclohex-3-en-1-ol **(35)**;
(1R,2R,5R,6S)-5,6-Isopropylidenedioxy-2-phenylcyclohex-3-en-1-ol **(36)**;
(1R,2S,5R,6S)-N-(5,6-Isopropylidenedioxy-2,4-diphenylcyclohex-3-enyl)-(4'-methylphenyl) sulfonamide **(37)**;
(1R,5R,6S)-N-(4,4-Diphenyl-5,6-isopropylidenedioxycyclohex-2-enyl)-(4'-methylphenyl) sulfonamide **(38)**;
(1R,2R,5R,6S)-N-[2,4-Di(cyclohexylmethyl)-5,6-isopropylidene-dioxycyclohex-3-enyl]-4'-methylphenyl)sulfonamide **(39);**
(1R,2R,5S,6S)-N-(4-Chloro-5,6-isopropylidenedioxy-2-methylcyclohex-3-enyl)-(4'-methylphenyl) sulfonamide **(40);**
(1R,4S,5R,6S)-N-(4-Cyclohexylmethyl-5,6-isopropylidene-dioxycyclohex-2-eny l)-4'-methylphenyl) sulfonamide **(41);**
(1R, 4R,5R,6S)-N-(5,6-Isopropylidenedioxy-4-phenylcyclohex-2-enyl)-(4'-methylphenyl) sulfonamide **(42);**
(1R,2R,5R,6S)-N-(5,6-Isopropylidenedioxy-2-phenylcyclohex-3-enyl)-(4'-methylphenyl) sulfonamide **(43);**
(1R,2R)-N-(6-Hydroxycyclohexa-2,4-dienyl)-(4'-methylphenyl)sulfonamide (24); and
(1R,2S,5R,6S)-N-(5,6-Isopropylidenedioxy-2-methylcyclohex-3-enyl)-(4'-methylphenyl)sulfonamide **(44).**

11. A compound useful as a synthon, having the formula 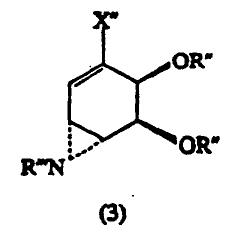 wherein:
X' is H, halogen, CN, alkyl, aryl, or a heteroatom;
each R" is independently any alcohol protecting group, provided that the R" at alcohols C2 and C3 may be the same or different; and
R"' is H, CBZ, tosylamide or any substituted or unsubstituted arylsulfonic acid amide, benzyl or CO₂Me.

12. The compound of claim 11 which is: (1R,4S,SS,6R)-3-Chloro-4,5-isopropylidenedioxy-7-(4'-methyl-phenyl)sulfonylbicyclo[4.1.0]hept-2-ene **(3a)**; (1R,4R,5S,6R)-3-Bromo-4,5-isopropylidenedioxy-7-(4'-methylphenyl)-sulfonylbicyclo[4.1.0]hept-2-ene **(3c)**; and 1R,4R,5S,6R)-4,5-Isopropylidenedioxy-7-(4'-methylphenyl)sulfonylbicyclo[4.1.0]hept-2-ene **(3b)**.

## Patentansprüche

1. Verfahren zur Herstellung einer gewünschten Verbindung, die ein Konjugat aus Einheiten ist, die aus Cyclitolen, Kohlenhydraten und C-Analogen davon ausgewählt sind, wobei das Verfahren umfasst:
das Koppeln eines Epoxids der Formel: 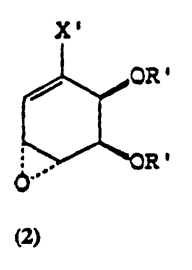
worin X' = H, Halogen, CN, Alkyl, Aryl oder ein Heteroatom ist; und jedes R' jeweils unabhängig eine geeignete Alkoholschutzgruppe ist, mit der Maßgabe, dass die R' an Alkohol C2 und C3 gleich oder voneinander verschieden sein können;
oder eines Aziridins der Formel: 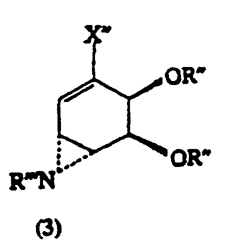 worin X" = H, Halogen, CN, Alkyl, Aryl oder ein Heteroatom ist; jedes R" jeweils unabhängig eine beliebige Alkoholschutzgruppe ist, mit der Maßgabe, dass die R" an den Alkoholen C2 und C3 gleich oder voneinander verschieden sein können; und R" = H, CBZ, Tosyl oder ein substituiertes oder unsubstituiertes Arylsulfonsäureamid, Benzyl oder CO₂Me ist;
unter geeigneten Bedingungen mit einem nukleophilen Cyclitol, Zucker oder C-Zucker mit einem nukleophilen Kohlenstoffatom oder Heteroatom, was zum Koppeln des Epoxids oder Aziridins mit dem Cyclitol, Zucker oder C-Zucker über eine Ringöffnungsreaktion führt, wodurch eine nukleophile Gruppe erzeugt wird, die für eine weitere Reaktion zur Verfügung steht.

2. Verfahren nach Anspruch 1, weiters umfassend den Schrift des Einsatzes der erzeugten nukleophilen Gruppe in einer weiteren Reaktion mit einem weiteren Epoxid (2) oder Aziridin (3).

3. Verfahren nach Anspruch 1, worin die gewünschte Verbindung der Formel (I): 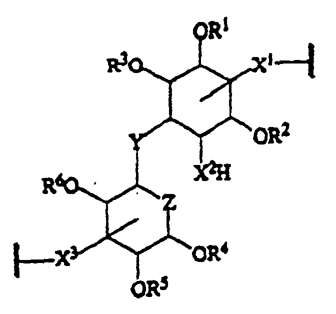 (1) entspricht,
worin X¹ = O ist, X² = O oder NR''' ist und X³ = O, NH, S oder CH₂ ist;
Y = O, CH₂, S oder NH ist;
Z = O, CH₂, S oder NH ist; und
R¹ bis R⁶ jeweils unabhängig H oder eine geeignete Alkoholschutzgruppe sind;
wobei das Verfahren umfasst:
a) das Koppeln eines Epoxids der Formel: 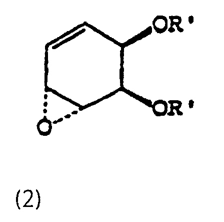 worin jedes R' jeweils unabhängig eine geeignete Alkoholschutzgruppe ist, mit der Maßgabe, dass die R' an Alkohol C2 und C3 gleich oder voneinander verschieden sein können;
oder eines Aziridins der Formel: 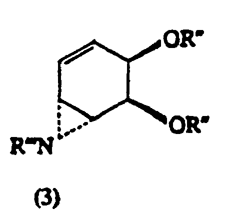 worin:
jedes R" jeweils unabhängig eine beliebige Alkoholschutzgruppe ist, mit der Maßgabe, dass die R" an den Alkoholen C2 und C3 gleich oder voneinander verschieden sein können; und R"' = H, CBZ, Tosyl oder ein substituiertes oder unsubstituiertes Arylsulfonsäureamid, Benzyl oder CO₂Me ist;
unter geeigneten Bedingungen mit einem nukleophilen Cyclitol, Zucker oder C-Zucker mit einem nukleophilen Kohlenstoffatom oder Heteroatom, was zur Kopplung des Elektrophils mit dem Cyclitol oder C-Zucker über eine Ringöffnungsreaktion des Elektrophils führt, wodurch eine nukleophile Gruppe erzeugt wird, die für eine weitere Reaktion zur Verfügung steht; und weiters umfassend:
(b) das Umwandeln der in der elektrophilen Gruppe vorhandenen Doppelbindung in das Diol oder das geschützte Diol (das OR¹ und OR² umfasst) der Struktur (I), und
(c) gegebenenfalls das Entfernen der Schutzgruppe von der Verbindung aus Schritt (b).

4. Verfahren nach Anspruch 3, weiters umfassend den Schritt des Einsatzes der erzeugten nukleophilen Gruppe in einer weiteren Reaktion mit einem weiteren Epoxid (2) oder Aziridin (3).

5. Verfahren nach Anspruch 3, worin das bzw. der nukleophile Cyclitol, Zucker oder C-Zucker ein Heteroatom- oder Kohlenstoffatom-Nukleophil ist, das den nachstehend dargestellten Abschnitt der Formel (1) umfasst: 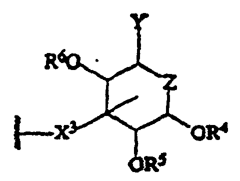 worin X³, Y, Z und R⁴ bis R⁶ wie oben definiert sind.

6. Verfahren zur Herstellung von Conduritol-Derivaten, umfassend das Koppeln eines Elektrophils der Formel (2), worin X' = H, Br oder Cl ist, mit einem Nukleophil, die ein organometallisches Reagens der Formel RMgBr, R₂CuLi, R₂CuCnLi₂ oder RSnMe₃/Pd(0) umfasst, worin R = Methyl, Methylcyclohexyl oder Phenyl ist, unter geeigneten Bedingungen.

7. Verfahren zur Herstellung von Conduritol-Derivaten, umfassend das Koppeln eines Elektrophils der Formel (3), worin X" = H, Br oder Cl ist, mit einem Nukleophil, das ein organometallisches Reagens der Formel RMgBr, R₂CuLi, R₂CuCnLi₂ oder RSnMe₃/Pd(0) umfasst, worin R = Methyl, Methylcyclohexyl oder Phenyl ist, unter geeigneten Bedingungen.

8. Verfahren nach Anspruch 1 oder 3, worin die gewünschte Verbindung aus der aus einem Cyclitol-Konjugat, einem Kohlehydrat-Konjugat und einem teilweise oder vollständig carbozyklischen Analog davon bestehenden Gruppe ausgewählt ist.

9. Verfahren nach Anspruch 1 oder 3, worin die gewünschte Verbindung GM3 (54), Silyl-Lewis X (56) oder ein Kohlenstoff- oder Heteroatom-Konjugat von einem davon ist.

10. Verfahren nach Anspruch 6 oder 7, worin das Conduritol-Derivat aus der aus:
(1R,2R,5S,6S)-(4-Chlor-5,6-isopropylidendioxy-2-methylcyclohex-3-en-1-ol (25);
(1R,2R,5S,6S)-(4-Chlor-5,6-isopropylidendioxy-2-cyclohexylmethylcyclohex-3-en-1-ol (26);
(1R,2R,5R,6S)-2,4-Di(cyclohexylmethyl)-5,6-isopropylidendioxy-cyclohex-3-en-1-ol (27);
(1R,5R,6S)-5,6-Isopropylidendioxy-4,4-diphenylcyclohex-2-en-1-ol (29);
(1R,2S,5R,6S)-5,6-Isopropylidendioxy-4-methylcyclohex-3-en-1-ol (30);
(1R,2S,5R,6S)-5,6-Isopropylidendioxy-2-methylcyclohex-3-en-1-ol (31);
(1R,4S,5R,6S)-4-Cyclohexylmethyl-5,6-isopropylidendioxycyclohex-2-en-1-ol (32);
(1R,2R,5R,6S)-2-Cyclohexylmethyl-5,6-isopropylidendioxycyclohex-3-en-1-ol (33);
(1R,2R,5R,6S)-5,6-lsopropylidendioxy-2-methylcyclohex-3-en-1-ol (35);
(1R,2R,5R,6S)-5,6-Isoproyöidendioxy-2-phenylcyclohex-3-en-1-ol (36);
(1R,2S,5R,6S)-N-(5,6-Isopropylidendioxy-2,4-diphenylcyclohex-3-enyl)-(4'-methylphenyl)sulfonamid (37);
(1R,5R,6S)-N-(4,4-Diphenyl-5,6-isopropylidendioxycyclohex-2-enyl)-(4'-methylphenyl)-sulfonamid (38);
(1R,2R,5R,6S)-N-[2,4-Di(cyclohexylmethyl)-5,6-isopropylidendioxycyclohex-3-enyl]-4'methylphenyl)sulfonamid (39);
(1R,2R,5S,6S)-N-(4-Chlor-5,6-isopropylidendioxy-2-methylcyclohex-3-enyl)-(4'-methylphenyl)sulfonamid (40);
(1R,4S,SR,6S)-N-(4-Cyclohexylmethyl-5,6-isopropylidendioxycyclohex-2-enyl)-4'methylphenyl)sulfonamid (41);
(1R,4R,5R,6S)-N-(5,6-Isopropylidendioxy-4-phenylcyclohex-2-enyl)-(4'-methylphenyl)-sulfonamid (42);
(1R,2R,5R,6S)-N-(5,6-Isopropylidendioxy-2-phenylcyclohex-3-enyl)-(4'-methylphenyl)-sulfonamid (43);
(1R,2R)-N-(6-Hydroxycyclohexa-2,4-dienyl)-(4'-methylphenyl)sulfonamid (24); und
1R,2S,5R,6S)-N-(5,6-Isopropylidendioxy-2-methylcyclohex-3-enyl)-(4'-methylphenyl)-sulfonamid (44);
bestehenden Gruppe ausgewählt ist.

11. Als Synthon nützliche Verbindung der Formel: 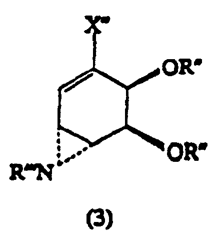 worin
X' = H, Halogen, CN, Alkyl, Aryl oder ein Heteroatom ist;
jedes R" jeweils unabhängig voneinander eine beliebige Alkoholschutzgruppe ist, mit der Maßgabe, dass die R" an den Alkoholen C2 und C2 gleich oder voneinander verschieden sein können; und
R"' = H, CBZ, Tosylamid oder ein beliebiges substituiertes oder unsubstituiertes Arylsulfonsäureamid, Benzyl oder CO₂Me ist.

12. Verbindung nach Anspruch 11, die:
(1R,4S,5S,6R)-3-Chlor-4,5-isopropylidendioxy-7-(4'-methylphenyl)sulfonylbicyclo[4.1.0]-hept-2-en (3a); (1R,4R,5S,6R)-3-Brom-4,5-isopropylidendioxy-7-(4'-methylphenyl)sulfonylbicyclo[4.1.0]hept-2-en (3c); und (1R,4R,5S,6R)-4,5-Isopropylidendioxy-7-(4'-methylphenyl)sulfonylbicyclo[4.1.0]hept-2-en (3b) ist.

## Revendications

1. Méthode de préparation d'un composé souhaité qui est un conjugué d'unités sélectionnées parmi des cyclitols, des carbohydrates et leurs analogues C, la méthode comprenant :
le couplage d'un époxyde de la formule : 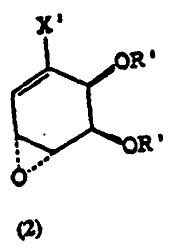
où
X' est H, halogène, CN, alkyle, aryle ou un hétéroatome ; et chaque R' est indépendamment tout groupe protecteur d'alcool approprié, à condition que R' à l'alcool C2 et C3 puisse être identique ou différent ;
ou une aziridine de la formule : 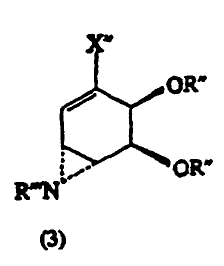 où
X" est H, halogène, CN, alkyle, aryle ou un hétéroatome ; chaque R" est indépendamment tout groupe protecteur d'alcool, à condition que R" aux alcools C2 et C3 puisse être identique ou différent ; et R"' est H, CBZ, tosyle ou tout amide d'acide arylsulfonique non substitué ou substitué, benzyle ou CO₂Me ;
en conditions appropriées, avec un cyclitol nucléophile, sucre ou sucre C ayant un atome de carbone nucléophile ou hétéroatome avec pour résultat le couplage de l'époxyde
ou de l'aziridine au cyclitol, au sucre, ou au sucre C via une réaction d'ouverture du cycle, pour ainsi générer un groupe nucléophile qui est disponible pour une plus ample réaction.

2. Méthode de la revendication 1, comprenant de plus l'étape d'employer le groupe nucléophile généré dans une autre réaction avec un autre époxyde (2) ou une autre aziridine (3).

3. Méthode de la revendication 1, où le composé souhaité est de la formule (1) : 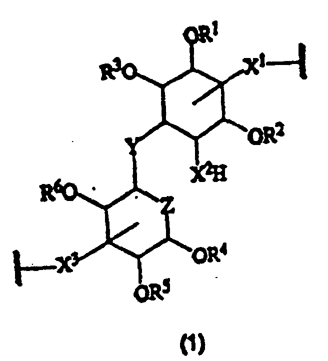 où
X¹ est O, X² est O ou NR"' et X³ est O, NH, S, ou CH₂ ;
Y est O, CH₂, S ou NH ;
Z est O, CH₂, NH ou S ; et
R¹-R⁶ indépendamment sont H ou tout groupe protecteur d'alcool approprié ;
la méthode comprenant :
a) le couplage d'un époxyde de la formule 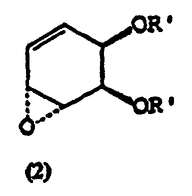 où
chaque R' est indépendamment tout groupe protecteur d'alcool approprié à condition que les R' à l'alcool C2 et C3 puissent être identiques ou différents ;
ou une aziridine de la formule : 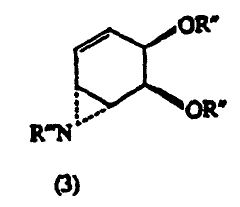 où
chaque R" est indépendamment tout groupe protecteur d'alcool, à condition que les R" aux alcools C2 et C3 puissent être identiques ou différents ; et R"' et H, CBZ, tosyle ou tout amide d'acide aryl sulfonique non substitué ou substitué, benzyle ou CO₂Me ;
en conditions appropriées, avec un cyclitol nucléophile, un sucre ou un sucre C ayant un atome de carbone nucléophile ou hétéroatome avec pour résultat le couplage de l'électrophile au cyclitol ou sucre C par une réaction d'ouverture du cycle dudit électrophile, pour ainsi générer un groupe nucléophile qui est disponible pour une plus ample réaction ; et comprenant de plus
(b) la conversion de la double liaison présente dans le groupe électrophile en diol ou diol protégé (comprenant OR¹ et OR³) de la structure (1), et
(c) facultativement, la suppression de la protection du composé de l'étape (b).

4. Méthode de la revendication 3, comprenant de plus l'étape d'employer le groupe nucléophile généré dans une autre réaction avec un autre époxyde (2) ou une autre aziridine (3).

5. Méthode de la revendication 3, où le cyclitol nucléophile, le sucre ou le sucre C est un hétéroatome ou atome de carbone nucléophile comprenant la portion de la formule (1) montrée ci-dessous : 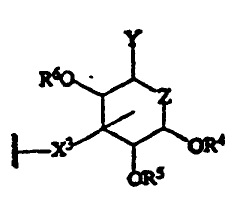 où X³, Y, Z et R⁴-R⁶ sont définis ci-dessus.

6. Méthode de préparation de dérivés de conduritol, comprenant le couplage d'un électrophile de la formule (2), où X' est H, Br ou Cl, avec un nucléophile comprenant un réactif organométallique de la formule RMgBr, R₂CuLi, R₂CuCnLi₂, ou RsnMe₃/Pd(0) où R est méthyle, méthylcyclohexyle ou phényle, en conditions appropriées.

7. Méthode de préparation de dérivés de conduritol, comprenant le couplage d'un électrophile de formule (3), où X" est H, Br ou Cl, avec un nucléophile comprenant un réactif organométallique de la formule RMgBr, R₂CuLi, R₂CuCnLi₂, ou RsnMe₃/Pd(0) où R est méthyle, méthylcyclohexyle ou phényle, en conditions appropriées.

8. Méthode de la revendication 1 ou de la revendication 3, où le composé souhaité est sélectionné dans le groupe consistant en un conjugué de cyclitol, un conjugué de carbohydrate et leur analogue à moitié ou totalement carbocyclique.

9. Méthode de la revendication 1 ou de la revendication 3, où le composé souhaité est GM3 (54), silyl Lewis X (56), ou un conjugué carbone ou hétéroatome de chacun.

10. Méthode de la revendication 6 ou 7, où le dérivé de conduritol est sélectionné dans le groupe consistant en :
(1R,2R,5S,6S)-(4-Chloro-5,6-isopropylidènedioxy-2-méthylcyclohex-3-én-1-ol (25) ;
(1R,2R,5S,6S)-(4-Chlpro-5,6-isopropylidènedioxy-2-cyclohexylméthylcyclohex-3-én-1-ol (26) ;
(1R,2R,5R,6S)-2,4-Di(cyclohexylméthyl)-5,6-isopropylidènedioxy-cyclohex-3-én-1-ol (27) ;
(1R,5R,6S)-5,6-Isopropylidènedioxy-4,4-diphénylcyclohex-2-én-1-ol (29) ;
(1R,2S,5R,6S)-5,6-Isopropylidènedioxy-4-méthylcyclohex-3-en-1-ol (30) ;
(1R,2S,5R,6S)-5,6-Isopropylidènedioxy-2-méthylcyclohex-3-én-1-ol (31) ;
(1R,4S,SR,6S)-4-Cyclohexylméthyl-5,6-isopropylidènedioxycyclohex-2-én-1-ol (32) ;
(1R,2R,5R,6S)-2-Cyclohexylméthyl-5,6-isopropylidènedioxycyclohex-3-én-1-ol (33) ;
(1R,2R,5R,6S)-5,6-Isopropylidènedioxy-2-méthylcyclohex-3-en-1-ol (35) ;
(1R,2R,5R,6S)-5,6-Isopropylid-nedioxy-2-phénylcyclohex-3-én-1-ol (36) ;
(1R,2S,5R,6S)-N-(5,6-Isopropylidènedioxy-2,4-diphénylcyclohex-3-ényl)-(4'-méthylphényl)sulfonamide (37) ;
(1R,5R,6S)-N-(4,4-Diphényl-5,6-isopropylidènedioxycyclohex-2-ényl)-(4'-méthylphényl) sulfonamide (38) ;
(1R,2R,5R,6S)-N-[2,4-Di(cyclohexylméthyl)-5,6-isopropylidène-dioxycyclohex-3-ényl]-4'-méthylphényl)sulfonamide (39) ;
(1R,2R,5S,6S)-N-(4-Chloro-5,6-isopropylidènedioxy-2-méthylcyclohex-3-ényl)-(4'-méthylphényl)sulfonamide (40) ;
(1R,4S,5R,6S)-N-(4-Cyclohexylméthyl-5,6-isopropylidène-dioxycyclohex-2-ényl))-4'-méthylphényl)sulfonamide (41) ;
(1R,4R,5R,6S)-N-(5,6-Isopropylidènedioxy-4-phénylcyclohex-2-ényl)-(4'-méthylphényl)sulfonamide (42) ;
(1R,2R,5R,6S)-N-(5,6-Isopropylidènedioxy-2-phénylcyclohex-3-ényl)-(4'-méthylphényl)sulfonamide (43) ;
(1R,2R)-N-(6-Hydroxycyclohexa-2,4-diényl)-(4'-méthylphényl)sulfonamide (24) ; et
(1R,2S,5R,6S)-N-(5,6-Isopropylidènedioxy-2-méthylcyclohex-3-ényl)-(4'-méthylphényl)sulfonamide (44).

11. Composé utile comme synthon, ayant la formule 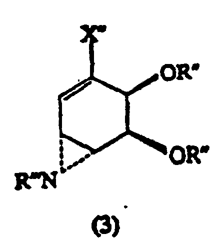 où
X' est H, halogène, CN, alkyle, aryle ou un hétéroatome ;
chaque R" est indépendamment tout groupe protecteur d'alcool, à condition que les R" aux alcools C2 et C3 puissent être identiques ou différents ; et
R"' est H, CBZ, tosylamide ou tout amide d'acide arylsulfonique non substitué ou substitué, benzyle ou CO₂Me.

12. Composé de la revendication 11 qui est :
(1R,4S,5S,6R)-3-Chloro-4,5-isopropylidènedioxy-7-(4'-méthyl-phényl)sulfonylbicyclo[4.1.0]hept-2-ène (3a) ;
(1R,4R,5S,6R)-3-Bromo-4,5-isopropylidènedioxy-7-(4'-méthylphényl)-sulfonylbicyclo[4.1.0]hept-2-ène (3c) ; et
1R,4R,5S,6R)-4,5-Isopropylidènedioxy-7-(4'-méthylphényl) sulfonylbicyclo[4.1.0]hept-2-ène (3b).
